(19) Europäisches Patentamt
European Patent Office
Office européen des brevets

(11) Veröffentlichungsnummer: 0 007 605 B1

# (12) EUROPÄISCHE PATENTSCHRIFT

(45) Veröffentlichungstag der Patentschrift: 15.09.82

(51) Int. Cl.³: C 07 C 93/06, C 07 D 263/14

(21) Anmeldenummer: 79102592.7

(22) Anmeldetag: 23.07.79

## (54) Verfahren zur Umkehrung der Konfiguration in optisch aktiven Verbindungen und dazu benötigte neue optisch aktive Zwischenprodukte und deren Salze.

(30) Priorität: 28.07.78 CH 8145/78

(43) Veröffentlichungstag der Anmeldung: 06.02.80 Patentblatt 80/3

(45) Bekanntmachung des Hinweises auf die Patenterteilung: 15.09.82 Patentblatt 82/37

(84) Benannte Vertragsstaaten: AT BE CH DE FR GB IT LU NL SE

(56) Entgegenhaltungen:
AT-B-331 791
CH-A-591 477
DE-A-1 543 684
DE-A-1 720 005
DE-A-2 322 053
DE-A-2 503 751
DE-A-2 559 755
DE-B-1 493 856
Chem. Abstr. 84, 43 593x (1976)

(73) Patentinhaber: CIBA-GEIGY AG, Patentabteilung Postfach, CH-4002 Basel (CH)

(72) Erfinder: Zergényi, Janos, Dr., Jurastrasse 31, CH-4411 Seltisberg (CH)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 007 605 B1

BUNDESDRUCKEREI BERLIN

## Verfahren zur Umkehrung der Konfiguration in optisch aktiven Verbindungen und dazu benötigte neue optisch aktive Zwischenprodukte und deren Salze

Die Erfindung betrifft ein Verfahren zur Umkehrung der Konfiguration in optisch aktiven Verbindungen der Formel

$$Ar_1-O-CH_2-\underset{}{\overset{\displaystyle OH}{\overset{|}{C}H}}-CH_2-NH-R_1 \qquad (I)$$

worin $Ar_1$ für einen gegebenenfalls substituierten mono- oder polycyclischen, carbo- oder heterocyclischen Rest steht, der mindestens einen Ring mit aromatischem Charakter aufweist, und der über ein Ringkohlenstoffatom, vorzugsweise des Rings mit aromatischem Charakter, mit dem Sauerstoffatom verbunden ist, $R_1$ einen gegebenenfalls substituierten aliphatischen, cycloaliphatischen oder araliphatischen Kohlenwasserstoffrest bedeutet, oder deren Salzen, sowie neue zur Durchführung des Verfahrens geeignete Zwischenprodukte und deren Salze.

Die erfindungsgemäße Konfigurationsumkehr bezieht sich auf die in der Gruppe

$$-O-CH_2-\overset{\displaystyle OH}{\overset{|}{C}H}-CH_2-NH-R_1$$

vorhandene sekundäre Alkoholgruppe, deren räumliche Anordnung entweder der R(+)- oder der S(−)-Konfiguration entspricht.

Dementsprechend wird nach dem erfindungsgemäßen Verfahren eine Verbindung der R(+)-Konfiguration in eine der S(−)-Konfiguration bzw. eine der S(−)-Konfiguration in eine solche der R(+)-Konfiguration umgewandelt. Durch diese Konfigurationsumkehr wird eine Umkehrung des optischen Drehsinns bewirkt.

Carbocyclische Reste $Ar_1$ aromatischen Charakters sind in erster Linie Phenyl, sowie gegebenenfalls teilweise gesättigte, bicyclische, aromatische Kohlenwasserstoffreste, wie Naphthyl, z. B. 1- oder 2-Naphthyl, 1,2,3,4-Tetrahydro-benznaphthyl, z. B. 1,2,3,4-Tetrahydro-5-naphthyl, benz-Idenyl, z. B. 4- oder 5-Idenyl, oder benz-Indanyl, z. B. 4- oder 5-Indanyl, ferner gegebenenfalls teilweise gesättigte polycyclische aromatische Kohlenwasserstoffreste, wie benz-Fluorenyl, z. B. 4-Fluorenyl, oder 9,10-Äthano-9,10-dihydro-benz-anthryl, z. B. 9,10-Äthano-9,10-dihydro-1-anthryl, wobei teilweise gesättigte Reste der obigen Art über ein Ringkohlenstoffatom des aromatischen Teils mit dem Sauerstoffatom verbunden sind.

Heterocyclische Reste $Ar_1$ enthalten als Ringheteroatome in erster Linie ein oder mehrere Ringstickstoffatome sowie, vorzugsweise zusätzlich zu einem Ringstickstoffatom, auch ein Ringsauerstoff- oder Ringschwefelatom. Solche Reste sind insbesondere monocyclische, fünf- oder sechsgliedrige, mono-, di- oder triazacyclische Reste, in erster Linie monocyclische, monoazacyclische, sechsgliedrige Reste aromatischen Charakters wie Pyridyl, z. B. 2-, 3- oder 4-Pyridyl, monocyclische, diazacyclische, sechsgliedrige Reste aromatischen Charakters, wie Pyridazinyl, z. B. 3-Pyridazinyl, Pyrimidinyl, z. B. 2- oder 4-Pyrimidinyl, oder Pyrazinyl, z. B. 2-Pyrazinyl, monocyclische, thiadiazacyclische fünfgliedrige Reste aromatischen Charakters, wie Thiadiazolyl, z. B. 1,2,5-Thiadiazol-3-yl, gegebenenfalls teilweise gesättigte, bicyclische, monoazacyclische Reste aromatischen Charakters mit einem fünf- oder sechsgliedrigen heterocyclischen Ring, wie Indolyl, z. B. 4-Indolyl, oder gegebenenfalls teilweise gesättigtes Chinolinyl, z. B. 1,2,3,4-Tetrahydro-5-chinolinyl, oder bicyclische monothiacyclische Reste teilweise aromatischen Charakters, wie 2H-Thiochromenyl, z. B. 2H-8-Thiochromenyl.

Die obigen Reste $Ar_1$ können unsubstituiert oder substituiert sein, wobei $Ar_1$ in erster Linie einen, aber auch mehrere, insbesondere zwei Substituenten enthält. Letztere sind in erster Linie gegebenenfalls substituierte aliphatische oder cycloaliphatische Kohlenwasserstoffreste, gegebenenfalls veräthertе oder veresterte Hydroxy- oder Mercaptogruppen, Acylreste, gegebenenfalls funktionell abgewandelte Carboxylgruppen, Nitro oder gegebenenfalls substituierte Aminogruppen. Gesättigte Teile der Gruppe $Ar_1$ können außer den oben angegebenen Substituenten auch zweifach gebundene Substituenten, in erster Linie Oxo enthalten.

Aliphatische Kohlenwasserstoffreste als Substituenten des Restes $Ar_1$ sind insbesondere Niederalkyl oder Niederalkenyl, ferner Niederalkinyl. Substituenten von solchen Resten, insbesondere von Niederalkyl sowie Niederalkenyl, sind gegebenenfalls veräthertes oder verestertes Hydroxy oder Mercapto, z. B. Niederalkoxy, Niederalkylthio oder Halogen, gegebenenfalls funktionell abgewandeltes Carboxyl, insbesondere gegebenenfalls N-substituiertes, wie N-niederalkyliertes Carbamoyl, oder gegebenenfalls substituiertes Amino, insbesondere Acylamino, worin Acyl den Rest einer organischen Carbonsäure oder eines Kohlensäurehalbderivates sowie einer organischen Sulfonsäure darstellt, wie Niederalkanoylamino, Niederalkoxycarbonylamino oder gegebenenfalls N-substituiertes wie N'-niederalkyliertes Ureido, z. B. Ureido, N'-Niederalkyl-ureido oder N',N'-Diniederalkyl-ureido, ferner

Niederalkylsulfonylamino. Substituierte Niederalkylreste sind in erster Linie Hydroxyniederalkyl, Niederalkoxy-niederalkyl, Niederalkylthio-niederalkyl, Halogen-niederalkyl, gegebenenfalls N-niederalkyliertes Carbamoylniederalkyl, Niederalkanoylaminoniederalkyl oder Niederalkoxycarbonylaminoniederalkyl, ferner Niederalkanoylamino-niederalkenyl oder Niederalkoxycarbonylamino-niederalkenyl.

Cycloaliphatische Kohlenwasserstoffreste werden insbesondere durch mono- sowie polycyclisches Cycloalkyl dargestellt.

Verätherte Hydroxy- oder Mercaptogruppen als Substituenten eines Restes $Ar_1$ sind in erster Linie durch gegebenenfalls substituierte aliphatische Kohlenwasserstoffreste veräthertes Hydroxy oder Mercapto, wie Niederalkoxy oder Phenylniederalkoxy, Niederalkenyloxy oder Niederalkinyloxy, ferner Tetrahydrofurfuryloxy, Niederalkylthio oder Niederalkenylthio. Substituenten von solchen veräthernden aliphatischen Kohlenwasserstoffresten, insbesondere von verätherndem Niederalkyl, sind in erster Linie gegebenenfalls veräthertes oder verestertes Hydroxy oder Mercapto, wie Niederalkoxy, Niederalkylthio oder Halogen, oder gegebenenfalls substituiertes Amino, wie Acylamino, z. B. Niederalkanoylamino oder Niederalkoxycarbonylamino. Durch entsprechend substituierte aliphatische Kohlenwasserstoffreste veräthertes Hydroxy oder Mercapto ist insbesondere Niederalkoxyniederalkoxy, Niederalkylthio-niederalkoxy oder Niederalkoxy-niederalkylthio, ferner Niederalkanoylamino-niederalkoxy oder Niederalkoxycarbonylamino-niederalkoxy.

Veresterte Hydroxy- oder Mercaptogruppen als Substituenten von Gruppen $Ar_1$ sind in erster Linie Halogen, ferner Niederalkanoyloxy.

Acylgruppen als Substituenten des Restes $Ar_1$ stellen in erster Linie Niederalkanoyl dar.

Gegebenenfalls funktionell abgewandelte Carboxylgruppen als Substituenten von $Ar_1$ sind insbesondere verestertes oder amidiertes Carboxyl, ferner Cyan. Verestertes Carboxyl ist in erster Linie Niederalkoxycarbonyl, während amidiertes Carboxyl gegebenenfalls substituiertes Carbamoyl, wie Carbamoyl, N-Niederalkyl-carbamoyl oder N,N-Diniederalkyl-carbamoyl darstellt.

Gegebenenfalls substituierte Aminogruppen als Substituenten von Gruppen $Ar_1$ sind insbesondere Acylamino, worin Acyl in erster Linie den entsprechenden Rest einer organischen Carbonsäure oder eines Halbderivates der Kohlensäure, ferner einer organischen Sulfonsäure darstellt, wie Niederalkanoylamino, Niederalkoxycarbonylamino oder gegebenenfalls N'-niederalkyliertes Ureido, oder N-Cycloalkyl-substituiertes Ureido, z. B. Ureido, N'-Niederalkyl-ureido oder N',N'-Diniederalkylureido oder N'-Cycloalkyl-ureido, ferner Niederalkylsulfonylamino sowie N-niederalkyliertes Amino, wie N-Niederalkylamino oder N,N-Diniederalkylamino, gegebenenfalls ungesättigtes N,N-Niederalkylenamino, N,N-Azaniederalkylenamino, N,N-Oxaniederalkylenamino oder N,N-Thianiederalkylenamino.

Aliphatische Kohlenwasserstoffreste $R_1$ sind in erster Linie Niederalkyl, insbesondere am Verknüpfungskohlenstoff verzweigtes Niederalkyl, ferner Niederalkenyl oder Niederalkinyl, während cycloaliphatische Kohlenwasserstoffreste insbesondere Cacloalkyl, inkl. polycyclisches Cycloalkyl, und araliphatische Kohlenwasserstoffreste in erster Linie Phenylniederalkyl darstellen. Substituenten solcher Kohlenwasserstoffreste sind z. B. für Niederalkyl veräthertes Hydroxy, insbesondere gegebenenfalls, z. B. durch funktionell abgewandeltes Carboxy, wie gegebenenfalls N-niederalkyliertes Carbamoyl, z. B. Carbamoyl, N-Niederalkyl-carbamoyl oder N,N-Diniederalkyl-carbamoyl, substituiertes Phenyloxy oder Pyridyloxy, oder gegebenenfalls funktionell abgewandeltes Carboxy, wie Carboxy, verestertes Carboxy, z. B. Niederalkoxycarbonyl, amidiertes Carboxy, wie gegebenenfalls N-niederalkyliertes Carbamoyl, z. B. Carbamoyl, N-Niederalkyl-carbamoyl oder N,N-Diniederalkyl-carbamoyl, oder Cyan, und z. B. für den aromatischen Teil von Phenylniederalkyl gegebenenfalls funktionell abgewandeltes Carboxyl, in erster Linie amidiertes Carboxy, wie gegebenenfalls durch Niederalkyl substituiertes Carbamoyl, wie N-Niederalkylcarbamoyl oder N,N-Diniederalkyl-carbamoyl, und/oder Hydroxy. In dieser Weise substituierte Niederalkylreste sind Phenyloxy-niederalkyl oder vorzugsweise gegebenenfalls N-alkyliertes Carbamoylphenyloxyniederalkyl, ferner Pyridyloxyniederalkyl oder vorzugsweise gegebenenfalls N-niederalkyliertes Carbamoylpyridyloxyniederalkyl, ferner Niederalkoxycarbonyl-niederalkyl, gegebenenfalls N-niederalkyliertes Carbamoyl-niederalkyl oder Cyan-niederalkyl.

Falls nicht besondere Angaben gemacht werden, enthalten die vorstehend sowie nachfolgend mit »nieder« bezeichneten Reste und Verbindungen vorzugsweise bis zu 7, einwertige Reste in erster Linie bis zu 5 und zweiwertige Reste 3 bis 6, in erster Linie 4 oder 5 Kohlenstoffatome.

Die vorstehend sowie nachfolgend verwendeten Allgemeinbegriffe haben, falls nicht besondere Angaben gemacht werden, vorzugsweise folgende Bedeutungen:

Niederalkyl ist z. B. Methyl, Äthyl, n-Propyl, Isopropyl, n-Butyl, Isobutyl, sek.-Butyl oder tert.-Butyl, ferner n-Pentyl, Isopentyl, Neopentyl, n-Hexyl, Isohexyl oder n-Heptyl. Am Verknüpfungskohlenstoffatom verzweigtes Niederalkyl ist in erster Linie Isopropyl oder tert.-Butyl.

Niederalkenyl ist in erster Linie Allyl, ferner Vinyl, 2-Methyl-allyl, 2-Butenyl oder 3,3-Dimethylallyl, während Niederalkinyl z. B. Äthinyl oder Propargyl ist.

Niederalkoxy ist z. B. Methoxy, Äthoxy, n-Propyloxy, Isopropyloxy, n-Butyloxy, Isobutyloxy oder tert.-Butyloxy.

Phenylniederalkoxy ist z. B. Benzyloxy.

Niederalkylthio ist z. B. Methylthio, Äthylthio, Isopropylthio oder n-Butylthio.

Halogen ist z. B. Chlor oder Brom, ferner Fluor sowie Jod.

Gegebenenfalls N-niederalkyliertes Carbamoyl ist z. B. Carbamoyl, ferner N-Niederalkyl-carbamoyl wie N-Methylcarbamoyl oder N-Äthyl-carbamoyl, oder N,N-Diniederalkylcarbamoyl, wie N,N-Dimethyl-carbamoyl oder N,N-Diäthyl-carbamoyl.

Niederalkanoylamino ist z. B. Formylamino, Acetylamino, Propionylamino, Butyrylamino oder Pivaloylamino.

Niederalkoxycarbonylamino ist z. B. Methoxycarbonylamino, Äthoxycarbonylamino oder tert.-Butyloxycarbonylamino, während N'-Niederalkyl-ureido und N',N'-Diniederalkyl-ureido z. B. N'-Methylureido, N'-Äthylureido, N',N'-Dimethylureido oder N',N'-Diäthylureido sind, während N'-Cycloalkylureido z. B. N'-Cyclopropylureido, N'-Cyclopentyl-ureido, N'-Cyclohexylureido oder N'-Cycloheptylureido darstellt.

Niederalkylsulfonylamino ist z. B. Methylsulfonylamino oder Äthylsulfonylamino.

Hydroxyniederalkyl ist z. B. Hydroxymethyl oder 1- oder 2-Hydroxyäthyl.

Niederalkoxy-niederalkyl ist z. B. Niederalkoxymethyl oder vorzugsweise 2-(Niederalkoxy)-äthyl, wie Methoxymethyl, Äthoxymethyl, 2-Methoxy-äthyl, 2-Äthoxyäthyl oder 2-Isopropyloxy-äthyl.

Niederalkylthio-niederalkyl ist z. B. Niederalkylthiomethyl oder insbesondere 2-(Niederalkylthio)-äthyl, z. B. Methylthiomethyl, Äthylthiomethyl, 2-Methylthioäthyl oder 2-Äthylthioäthyl.

Halogen-niederalkyl ist insbesondere Trifluormethyl.

Gegebenenfalls N-niederalkyliertes Carbamoylniederalkyl ist z. B. Carbamoylmethyl oder 1- oder 2-Carbamoyläthyl, ferner N-Niederalkyl-carbamoyl-niederalkyl, wie N-Methylcarbamoylmethyl oder 1- oder 2-N-Methylcarbamoyl-äthyl, oder N,N-Diniederalkyl-carbamoyl-niederalkyl, wie N,N-Dimethylcarbamoyl-methyl oder 1- oder 2-N, N-Dimethylcarbamoyl-äthyl.

Niederalkanoylamino-niederalkyl ist z. B. Niederalkanoylaminomethyl oder vorzugsweise 2-Niederalkanoylaminoäthyl, wie Acetylaminomethyl, Propionylaminomethyl, 2-Acetylaminoäthyl, 2-Propionylamino-äthyl oder 2-Pivaloylamino-äthyl, während Niederalkoxycarbonylamino-niederalkyl z. B. Niederalkoxycarbonylamino-methyl oder vorzugsweise 2-Niederalkoxycarbonylamino-äthyl, wie Methoxycarbonylaminomethyl, Äthoxycarbonylaminomethyl, 2-Methoxycarbonylamino-äthyl, 2-Äthoxycarbonylamino-äthyl oder 2-tert.-Butyloxycarbonylamino-äthyl darstellt.

Niederalkanoylamino-niederalkenyl ist insbesondere 2-Niederalkanoylamino-vinyl, z. B. 2-Acetylamino-vinyl, 2-Propionylamino-vinyl oder 2-Pivaloylamino-vinyl, während Niederalkoxycarbonylamino-niederalkenyl vorzugsweise für 2-Niederalkoxycarbonylamino-vinyl, wie 2-Methoxycarbonylamino-vinyl, 2-Äthoxycarbonylamino-vinyl oder 2-tert.-Butyloxycarbonylamino-vinyl steht.

Cycloalkyl, inkl, polycyclisches Cycloalkyl, enthält vorzugsweise 3—10 Ringkohlenstoffatome und bedeutet Cyclopropyl, oder insbesondere Cyclopentyl, Cyclohexyl, oder Cycloheptyl, ferner Adamantyl, wie 1-Adamantyl.

Niederalkenyloxy ist insbesondere Allyloxy sowie 2-Methylallyloxy, ferner Vinyloxy, 2-Butenyloxy oder 3,3-Dimethylallyloxy, während Niederalkinyloxy z. B. für Propargyloxy steht.

Niederalkenylthio ist z. B. Allylthio, ferner 2-Methylallylthio oder 2-Butenylthio.

In einem Niederalkoxy-niederalkoxyrest sind die beiden Sauerstoffatome vorzugsweise durch mindestens 2, z. B. durch 2—3 Kohlenstoffatome getrennt; solche Reste sind deshalb z. B. Methoxymethoxy oder Äthoxymethoxy, in erster Linie jedoch 2-(Niederalkoxy)-äthoxy, z. B. Methoxyäthoxy oder 2-Äthoxy-äthoxy, sowie 3-(Niederalkoxy)-propyloxy, z. B. 3-Methoxy-propyloxy oder 3-Äthoxypropyloxy.

In einem Niederalkylthio-niederalkoxyrest sind das Schwefel- und das Sauerstoffatom vorzugsweise durch mindestens 2, z. B. durch 2—3 Kohlenstoffatome voneinander getrennt; solche Reste sind deshalb in erster Linie 2-(Niederalkylthio)-äthoxy, z. B. 2-Methylthio-äthoxy oder 2-Äthylthio-äthoxy.

Desgleichen sind in einem Niederalkoxy-niederalkylthiorest das Sauerstoff- und das Schwefelatom vorzugsweise durch mindestens 2, z. B. durch 2—3 Kohlenstoffatome voneinander getrennt; solche Reste sind in erster Linie 2-(Niederalkoxy)-äthylthio, z. B. 2-Methoxy-äthylthio oder 2-Äthoxy-äthylthio.

In Niederalkanoylamino-niederalkoxy- und Niederalkoxycarbonylamino-niederalkoxyresten sind das Stickstoff- und das Verknüpfungssauerstoffatom vorzugsweise durch mindestens 2, z. B. durch 2—3 Kohlenstoffatome voneinander getrennt; diese Reste sind in erster Linie 2-Niederalkanoylaminoäthoxy, z. B. 2-Acetylamino-äthoxy, 2-Propionylamino-äthoxy oder 2-Pivaloylamino-äthoxy, oder 2-Niederalkoxycarbonylaminoäthoxy, z. B. 2-Methoxycarbonylamino-äthoxy oder 2-Äthoxycarbonyläthoxy.

Niederalkanoyl ist insbesondere Acetyl, Propionyl oder Pivaloyl.

Niederalkanoyloxy ist z. B. Acetyloxy, Propionyloxy oder Pivaloyloxy.

Niederalkoxycarbonyl ist z. B. Methoxycarbonyl, Äthoxycarbonyl oder tert.-Butyloxycarbonyl.

N-Niederalkylamino oder N,N-Diniederalkylamino sind z. B. Methylamino, Äthylamino, Dimethylamino oder Diäthylamino.

Gegebenenfalls ungesättigtes N,N-Niederalkylenamino enthält vorzugsweise 5—7 Ringglieder und ist insbesondere Pyrrolidino oder Piperidino, ferner 1-Pyrryl, während N,N-Azaniederalkylenamino,

N,N-Oxaniederalkylenamino und N,N-Thianiederalkylenamino vorzugsweise 6 Ringglieder enthalten, wobei das zweite Ringheteroatom vom Verknüpfungsstickstoffatom durch 2 Kohlenstoffatome getrennt und im N,N-Azaniederalkylenaminorest gegebenenfalls, z. B. durch Niederalkyl, substituiert ist; solche Reste sind z. B. 4-Methyl-1-piperidino, 4-Morpholino oder 4-Thiomorpholino.

Phenylniederalkyl ist z. B. Benzyl 2- oder 2-Phenyläthyl.

Pyridyloxy ist z. B. 2-Pyridyloxy, 3-Pyridyloxy oder 4-Pyridyloxy.

In einem, vorzugsweise gegebenenfalls N-niederalkyliertes Carbamoyl als Substituenten enthaltenden Phenyloxyniederalkyl- und Pyridyloxy-niederalkylrest $R_1$ sind das Sauerstoffatom und das mit dem Stickstoffatom verbundene Verknüpfungskohlenstoffatom vorzugsweise durch mindestens 2, z. B. durch 2—3 Kohlenstoffatome voneinander getrennt. Solche Substituenten sind insbesondere 2-(gegebenenfalls N-niederalkyliertes Carbamoyl-phenyloxy)-niederalkyl, z. B. 2-(2-Carbamoylphenyloxy)-äthyl, 2-(4-Carbamoylphenyloxy)-äthyl, 2-(2-N-Methylcarbamoyl-phenyloxy)-äthyl oder 2-(4-N,N-Dimethylcarbamoyl-phenyloxy)-äthyl, ferner 2-(gegebenenfalls N-niederalkyliertes Carbamoylpyridyloxy)-niederalkyl, z. B. 2-(4-Carbamoyl-2-pyridyloxy)-äthyl, 2-(2-Carbamoyl-4-pyridyloxy)-äthyl oder 2-(3-Carbamoyl-2-pyridyloxy)-äthyl.

Niederalkoxycarbonyl-niederalkyl ist z. B. Niederalkoxycarbonylmethyl oder 1-Niederalkoxycarbonyl-2-propyl, z. B. Methoxycarbonylmethyl, Äthoxycarbonylmethyl, 1-Methoxycarbonyl-2-propyl oder 1-Äthoxycarbonyl-2-propyl.

Gegebenenfalls N-niederalkyliertes Carbamoylniederalkyl ist in erster Linie Carbamoylmethyl, ferner N-Niederalkylcarbamoyl-methyl oder N,N-Diniederalkylcarbamoyl-methyl, z. B. N-Methylcarbamoylmethyl, N-Äthylcarbamoylmethyl, N,N-Dimethylcarbamoylmethyl oder N,N-Diäthylcarbamoylmethyl, sowie 1-Carbamoyl-2-propyl, ferner 1-N-Niederalkyl-carbamoyl-2-propyl oder 1-N,N-Diniederalkyl-carbamoyl-2-propyl, z. B. 1-N-Methylcarbamoyl-2-propyl, 1-N-Äthylcarbamoyl-2-propyl, 1-N,N-Dimethylcarbamoyl-2-propyl oder 1-N,N-Diäthylcarbamoyl-2-propyl.

Cyan-niederalkyl ist z. B. Cyanmethyl oder 1-Cyan-2-propyl.

Vorzugsweise bedeutet in den Verbindungen der Formel I die Gruppe $Ar_1$ Napthyl, z. B. 1-Naphthyl, 5,6,7,8-Tetrahydronaphthyl z. B. 1-(5,6,7,8-Tetrahydro-6,7-dihydroxy-naphthyl, Fluorenyl, z. B. 4-Fluorenyl, 9,10-Äthano-9,10-dihydro-anthryl, z. B. 9,10-dihydro-1-anthryl, Indolyl, z. B. 4-Indolyl, 2H-Thiochromenyl, z. B. 2H-8-Thiochromenyl, Niederalkyl-phenyl, wie Methyl-phenyl, z. B. 2- oder 3-Methyl-phenyl, Halogen-niederalkyl-phenyl, wie Chlor-methyl-phenyl, z. B. 2-Chlor-5-methyl-phenyl, Niederalkenyl-phenyl, wie Allylphenyl, z. B. 2-Allylphenyl, Niederalkinylphenyl, wie Äthinyl-phenyl, z. B. 2-Äthinylphenyl, Cycloalkylphenyl, z. B. 2-Cyclopropyl-phenyl oder 2-Cyclopentyl-phenyl, Hydroxyphenyl, z. B. 4-Hydroxyphenyl, Acetoxy-trimethylphenyl z. B. 4-Acetoxy-2,3,5-trimethylphenyl, Hydroxyniederalkyl-phenyl, z. B. 2-Hydroxymethylphenyl, Niederalkoxyniederalkyl-phenyl, wie Niederalkoxymethylphenyl oder (2-Niederalkoxy-äthyl)-phenyl, z. B. 2-Methoxymethyl-phenyl oder 4-(2-Methoxyäthyl)-phenyl, Carbamoylniederalkyl-phenyl, z. B. Carbamoylmethyl-phenyl, Niederalkoxycarbonylaminoniederalkyl-phenyl, wie (2-Niederalkoxycarbonylamino-äthyl)-phenyl, z. B. 4-(2-Methoxycarbonylamino-äthyl)-phenyl, Halogen-niederalkoxycarbonylaminoniederalkyl-pyridyl, z. B. 3-Chlor-5-(2-methoxycarbonylaminoniederalkyl)-pyridyl, wie 3-Chlor-5-(2-methoxycarbonylamino-äthyl)-2-pyridyl, Niederalkoxycarbonylaminoniederalkenyl-phenyl, insbesondere (2-Niederalkoxycarbonylamino-vinyl)-phenyl, z. B. 4-(2-Methoxycarbonylaminovinyl)-phenyl, Niederalkoxy-phenyl, wie Methoxyphenyl, z. B. 2-Methoxy-phenyl, Phenylniederalkoxy-phenyl, wie Benzyloxy-phenyl, z. B. 4-Benzyloxyphenyl, Niederalkenyloxy-phenyl, wie Allyloxy-phenyl, z. B. 2-Allyloxy-phenyl, oder Methallyloxy-phenyl, z. B. 2-(2-Methylallyloxy)-phenyl, Niederalkinyloxy-phenyl, wie Propargyloxy-phenyl, z. B. 2-Propargyloxy-phenyl, Niederalkylthio-niederalkoxy-phenyl, worin das Schwefel- vom Sauerstoffatom durch 2—3 Kohlenstoffatome getrennt ist, wie (2-Niederalkylthio-äthoxy)-phenyl, z. B. 4-(2-Methylthio-äthoxy)-phenyl, Niederalkylthio-phenyl, wie Methylthio-phenyl, z. B. 2-Methylthiophenyl, Halogen-phenyl, wie Chlor-phenyl, z. B. 2-Chlor-phenyl, Niederalkanoyl-niederalkanoylamino-phenyl, z. B. 2-Acetyl-4-n-butyrylamino-phenyl, Cyan-phenyl, z. B. 2-Cyan-phenyl, Niederalkanoylamino-phenyl, wie Acetylamino-phenyl, z. B. 4-Acetylamino-phenyl, Ureido-phenyl wie 4-(N'-Methylureido)-phenyl oder 4-(N'-Cycloalkylureido)-phenyl, wie 4-(N'-Cyclohexylureido)-phenyl, Niederalkylsulfonylamino-phenyl, z. B. 4-Methylsulfonylamino-phenyl, (1-Pyrryl)-phenyl, z. B. 2-(1-Pyrryl)-phenyl, Morpholinothiadiazolyl, z. B. 4-Morpholino-1,2,5-thiadiazol-3-yl, Oxo-5,6,7,8-tetrahydro-benz-naphthyl, z. B. 5-Oxo-5,6,7,8-tetrahydro-1-naphthyl, oder Oxo-1,2,3,4-tetrahydro-benz-chinolinyl, z. B. 2-Oxo-1,2,3,4-tetrahydro-5-chinolinyl, Pyridyl z. B. 2-Pyridyl, Cyan-pyridyl z. B. 3-Cyan-2-pyridyl, Niederalkoxycarbonylaminoniederalkylpyridyl z. B. 5-(Äthoxycarbonylaminoäthyl)-2-pyridyl, Halogenpyridyl wie Chlor-pyridyl z. B. 3-Chlor-2-pyridyl, Niederalkoxypyridyl wie Äthoxy-pyridyl z. B. 3-Äthoxy-2-pyridyl, Pyrazinyl z. B. 2-Pyrazinyl, Niederalkylthio-pyrazinyl z. B. 3-Äthylthio-2-pyrazinyl oder Pyrimidinyl z. B. 2-Pyrimidinyl, ferner gegebenenfalls N-Niederalkyl-substituiertes Benzimidazolyl, wie 4-Benzimidazolyl, oder 1-Niederalkyl-benzimidazol-4-yl, wie 1-Methyl-benzimidazol-4-yl, 2,3-Dihydro-3-oxo-4H-benz[5,6]oxazinyl z. B. 2,3-Dihydro-3-oxo-4H-benz[5,6]oxazin-8-yl, 4H-1,3-benzoxazin-2(1H)-on-yl, z. B. 7-(4H-1,3-benz-oxazin-2(1H)-on)-yl, 2-(3H)-Benzoxazolon-yl, z. B. 7-(2-(3H)-benzoxazolon)-yl, 3,4-Dihydro-1H-chinazolin-2-on-yl z. B. 5-(3,4-Dihydro-1H-chinazolin-2-on)-yl, Tetrahydrofurfuryloxy-phenyl z. B. 2-Tetrahydrofurfuryloxy-phenyl und $R_1$ ist in erster Linie Niederalkyl, insbesondere am Verknüpfungskohlenstoff verzweigtes Niederalkyl, z. B. Isopropyl oder tert.-Butyl,

ferner Carbamoylphenyloxy-niederalkyl, wie 2-Carbamoylphenyloxy-niederalkyl, z. B. 2-(4-Carbamoylphenyloxy)-äthyl.

Verbindungen der obigen Formel I sind in erster Linie die folgenden, wobei Niederalkyl $R_1$ am Verknüpfungskohlenstoffatom verzweigt ist: 1-(Naphthyloxy)-3-niederalkylamino-2-propanol, z. B. 3-Isopropylamino-1-(1-naphthyloxy)-2-propanol, 1-(6,7-Dihydroxy-5,6,7,8-tetrahydro-1-naphthyloxy)-3-niederalkylamino-2-propanol, z. B. 3-tert.-Butylamino-1-(6,7-dihydroxy-5,6,7,8-tetrahydro-1-naphthyloxy)-2-propanol, 1-(benz-Fluorenyloxy)-3-niederalkylamino-2-propanol, z. B. 1-(4-Fluorenyloxy)-3-isopropylamino-2-propanol oder 3-tert.-Butylamino-1-(4-fluorenyloxy)-2-pivaloyloxy-propan, 1-(9,10-Äthano-9,10-dihydrobenz-anthryloxy)-3-niederalkylamino-2-propanole, z. B. 1-(9,10-Äthano-9,10-dihydro-1-anthryloxy)-3-isopropylamino-2-propanol, 1-(benz-Indolyloxy)-3-niederalkylamino-2-propanole, z. B. 1-(4-Indolyloxy)-3-isopropylamino-2-propanol, 3-Niederalkylamino-1-(2H-benz-thiochromenyloxy)-2-propanole, z. B. 3-tert.-Butylamino-1-(2H-8-thiochromenyloxy)-2-propanol, 3-Niederalkylamino-1-(niederalkylphenyloxy)-2-propanole, worin der Phenylrest zusätzlich mit Halogen substituiert sein kann, z. B. 3-Isopropylamino-1-(3-methyl-phenyloxy)-2-propanol, 1-(2-Chlor-5-methyl-phenyloxy-3-isopropylamino-2-propanol oder 3-tert.-Butylamino-1-(2-chlor-5-methyl-phenyloxy)-2-propanol, 3-(Carbamoylphenyloxyniederalkylamino)-1-(niederalkylphenyloxy-2-propanole, z. B. 3-[2-(4-Carbamoylphenyloxy)-äthylamino]-1-(2-methyl-phenyloxy)-2-propanol, 1-(Niederalkenyl-phenyloxy)-3-niederalkylamino-2-propanole, z. B. 1-(2-Allylphenyloxy)-3-isopropylamino-2-propanol, 1-(Niederalkinylphenyloxy)-3-niederalkylamino-2-propanole, z. B. 1-(2-Äthinyl-phenyloxy)-3-isopropylamino-2-propanol, 1-(Cycloalkyl-phenyloxy)-3-niederalkylamino-2-propanole, z. B. 1-(2-Cyclopropyl-phenyloxy)-3-isopropylamino-2-propanol oder 3-tert.-Butylamino-1-(2-cyclopentyl-phenyloxy)-2-propanol, 1-(Hydroxy-phenyloxy)-3-niederalkylamino-2-propanole, z. B. 1-(4-Hydroxy-phenyloxy)-3-isopropylamino-2-propanol, 1-(Hydroxyniederalkyl-phenyloxy)-3-niederalkylamino-2-propanole, z. B. 1-(2-Hydroxymethyl-phenyloxy)-3-isopropylamino-2-propanol, 1-Acetoxy-trimethylphenyloxy-3-niederalkylamino-2-propanol, z. B. 1-(4-Acetoxy-2,3,5-trimethylphenyloxy)-3-isopropylamino-2-propanol, 1-(Niederalkoxyniederalkylphenyloxy)-3-niederalkylamino-2-propanole, z. B. 3-Isopropylamino-1-(2-methoxymethyl-phenyloxy)-2-propanol, 3-tert.-Butylamino-1-(2-methoxymethylphenyloxy)-2-propanol oder 3-Isopropylamino-1-[4-(2-methoxyäthyl)-phenyloxy]-2-propanol, 1-(Carbamoylniederalkyl-phenyloxy)-3-niederalkylamino-2-propanole, z. B. 1-(4-Carbamoylmethyl-phenyloxy)-3-isopropylamino-2-propanol, 1-(Niederalkoxycarbonylaminoniederalkyl-phenyloxy)-3-niederalkylamino-2-propanole, z. B. 3-Isopropylamino-1-[4-(2-methoxycarbonylamino-äthyl)-phenyloxy]-2-propanol, 1-(Halogen-niederalkoxycarbonylaminoniederalkyl-pyridyloxy)-3-niederalkylamino-2-propanole, z. B. 1-[3-Chlor-4-(2-methoxycarbonylamino-äthyl)-2-pyridyloxy]-3-isopropylamino-2-propanol, 1-(Niederalkoxycarbonylamino-vinyl-phenyloxy)-3-niederalkylamino-2-propanole, z. B. 3-Isopropylamino-1-[4-(2-methoxycarbonylamino-vinyl)-phenyloxy]-2-propanol, 1-(Phenylniederalkoxy-phenyloxy)-3-niederalkylamino-2-propanole, z. B. 1-(4-Benzyloxy-phenyloxy)-3-isopropylamino-2-propanol, 1-(Niederalkoxy-phenyloxy)-3-niederalkylamino-2-propanole, z. B. 3-Isopropylamino-1-(2-methoxy-phenyloxy)-2-propanol, 1-(Niederalkenyloxyphenyloxy)-3-niederalkylamino-2-propanole, z. B. 1-(2-Allyloxyphenyloxy)-3-isopropylamino-2-propanol, 1-(2-Allyloxy-phenyloxy)-3-tert.-butylamino-2-propanol oder 3-Isopropylamino-1-(2-methylallyloxy-phenyloxy)-2-propanol, 1-(Niederalkinyloxy-phenyloxy)-3-niederalkylamino-2-propanole, z. B. 3-Isopropylamino-1-(2-propargyloxy-phenyloxy)-2-propanol, 3-Niederalkylamino-1-(niederalkylthioniederalkoxy-phenyloxy)-2-propanole, worin das Schwefel- vom Sauerstoffatom durch 2—3 Kohlenstoffatome getrennt ist, z. B. 3-Isopropylamino-1-[4-(2-methylthioäthoxy)-phenyloxy]-2-propanol, 1-(Halogen-phenyloxy)-3-niederalkylamino-2-propanole, z. B. 3-tert.-Butylamino-1-(2-chlor-phenyloxy)-2-propanol, 1-(Niederalkylthio-phenyloxy)-3-niederalkylamino-2-propanole, z. B. 3-Isopropylamino-1-(2-methylthio-phenyloxy)-2-propanol, 1-(Niederalkanoyl-niederalkanoylamino-phenyloxy)-3-niederalkylamino-2-propanol, z. B. 1-(2-Acetyl-4-n-butyrylamino-phenyloxy)-3-isopropylamino-2-propanol, 1-(Cyan-phenyloxy)-3-niederalkylamino-2-propanole, z. B. 3-tert.-Butylamino-1-(2-cyan-phenyloxy)-2-propanol oder 1-(2-Cyan-phenyloxy)-3-isopropylamino-2-propanol, 1-(Niederalkanoylamino-phenyloxy)-3-niederalkylamino-2-propanole, z. B. 1-(4-Acetylamino-phenyloxy)-3-isopropylamino-2-propanol, 1-(N'-Niederalkyl-ureido-N-phenyloxy)-3-niederalkylamino-2-propanole z. B. 1-(N'-Methyl-ureido-N-phenyloxy)-3-isopropylamino-2-propanol, 1-(N'-Cycloalkyl-ureido-phenyloxy)-3-niederalkylamino-2-propanole z. B. 1-(N'-Cyclohexyl-ureido-phenyloxy)-3-tert.-butylamino-2-propanol, 3-Niederalkylamino-1-(niederalkylsulfonylamino-phenyloxy)-2-propanole, z. B. 3-Isopropylamino-1-(4-methylsulfonylamino-phenyloxy)-2-propanol, 3-Niederalkylamino-1-(1-pyrryl)-phenyloxy-2-propanole, z. B. 3-Isopropylamino-1-[2-(1-pyrryl)]-phenyloxy-2-propanol, 1-(Morpholino-thiadiazolyloxy)-3-niederalkylamino-2-propanol, z. B. 3-Isopropylamino-1-(4-morpholino-1,2,5-thiadiazol-3-yloxy)-2-propanol, 3-Niederalkylamino-1-(oxo-5,6,7,8-tetrahydro-benz-naphthyloxy)-2-propanole, z. B. 3-tert.-Butyl-1-(5-oxo-5,6,7,8-tetrahydro-1-naphthyloxy)-2-propanol, oder 3-Niederalkylamino-1-(oxo-1,2,3,4-tetrahydro-benz-chinolinyloxy)-2-propanole, z. B. 3-Isopropylamino-1-(2-oxo-1,2,3,4-tetrahydro-5-chinolinyloxy)-2-propanol oder 3-tert.-Butylamino-1-(2-oxo-1,2,3,4-tetrahydro-5-chinolinyloxy)-2-propanol, 3-Niederalkylamino-1-(2-pyrazinyloxy)-2-propanole, z. B. 3-Isopropylamino-1-(2-pyrazinyloxy)-2-propanol, 3-Niederalkylamino-1-(3-niederalkylthio-2-pyrazinyloxy)-2-propanol z. B. 3-tert.-Butylamino-1-(3-äthylthio-2-pyrazinyloxy)-2-propanol, 3-Niederalkylamino-1-(2-pyrimidinyloxy)-2-propanole, z. B. 3-Isopropylamino-1-(2-pyrimidinyloxy)-2-propanol, (3-Niederalkylamino-2-hydroxy-propoxy)-benzimidazol-2-one, z. B. 4-(3-tert.-Butylamino-2-hydroxy-propoxy)-benzimidazol-2-on, 4-[3-(Hydroxyphenoxy)-alkylamino]-2-hydroxy-propoxy]-benzimidazol-2-on, z. B. 4-[3-[2-(4-Hydroxyphenoxy)-äthylamino]-2-hydroxy-propoxy]-benzimidazol-2-on, (3-Niederalkylamino-2-hydroxy-propoxy)-2,3-dihydro-(4H)-benz[5,6]oxazin-3-one z. B. 8-(3-tert.-Butylamino-2-hydroxy-propoxy)-2,3-dihydro-4H-benz[5,6]oxazin-3-on, (3-Niederalkylamino-2-hydroxy-propoxy)-3,4-dihydro-1H-chinazolin-2-one z. B. 5-(3-tert.-Butylamino-2-hydroxy-propoxy)-3,4-dihydro-1H-chinazolin-2-on, (3-Niederalkylamino-2-hydroxy-propoxy)-2(3H)-benzoxazolone, z. B. 7-(3-tert.-Butylamino-2-hydroxy-propoxy)-2(3H)-benzoxazolon, (3-Niederalkylamino-2-hydroxy-propoxy)-4H-1,3-benzoxazin-2(1H)-one z. B. 7-(3-tert.-Butylamino-2-hydroxy-propoxy)-4H-1,3-benzoxazin-2(1H)-on, 3-tert.-Butylamino-1-[2-(tetrahydrofurfuryloxy)-phenoxy]-2-propanol, 1-(Cyanpyridyloxy)-3-niederalkylamino-2-propanole, z. B. 3-tert.-Butylamino-1-(3-cyan-pyridyl-2-oxy)-2-propanol, 1-(Halogen-pyridyloxy)-3-niederalkylamino-2-propanole z. B. 3-Isopropylamino-1-(3-chlor-pyridyl-2-oxy)-2-propanol, 1-(Niederalkoxy-pyridyloxy)-3-niederalkylamino-2-propanole z. B. 3-tert.-Butylamino-1-(3-äthoxy-pyridyl-2-oxy)-2-propanol, 1-(Niederalkoxycarbonylaminoniederalkyl-pyridyloxy)-3-niederalkylamino-2-propanol z. B. 3-Isopropylamino-1-[5-(2-Äthoxycarbonylaminoäthyl)-pyridyl-2-oxy]-2-propanol, 1-(Niederalkoxycarbonylaminoniederalkylhalogen-pyridyloxy)-3-niederalkylamino-2-propanol z. B. 3-Isopropylamino-1-[5-(2-Äthoxycarbonylaminoäthyl)-3-chlor-pyridyl-2-oxy]-2-propanol und deren Salze.

Aus der deutschen Patentschrift Nr. 585 164 ist ein Verfahren zur Umkehrung des Drehsinns von optisch aktiven Phenylalkylaminen bekannt, indem man z. B. d-m-Oxyphenyläthanolmethylamin mit Essigsäureanhydrid und konz. Schwefelsäure unter Rückfluß erhitzt, den nach Abdampfen des überschüssigen Essigsäureanhydrid verbleibenden Rückstand mit verdünnter Schwefelsäure hydrolysiert und aus dem Hydrolysat das l-m-Oxyphenyläthanolmethylamin isoliert.

Nagai und Kanao [Liebig's Annalen der Chemie 470, 157—182 (1929)], besonders auf Seite 160, geben an, daß aus N-Benzoyl-nor-d,l-ephedrin mittels konz. Schwefelsäure unter Ringschluß Oxazolin entsteht, welches durch Kochen mit verdünnter Salzsäure hydrolysiert wird

In der US-Patentschrift Nr. 2 446 192 wird die Umwandlung von d,l-Allothreonin in d,l-Threonin beschrieben, wobei z. B. N-Acetyl- oder N-Benzoyl-d,l-allotheonin in Form des Methyl- oder Äthylesters mittels Thionylchlorid in das entsprechende Oxazolin umgewandelt und dieses mittels sauerer oder alkalischer Hydrolyse in d,l-Threonin überführt wird. Anstelle von Thionylchlorid werden als dehydratisierend und cyclisierend wirkende Mittel Phoshorpentachlorid, Phosphortrichlorid oder Phosphorpentoxid erwähnt, wobei Thionylchlorid bevorzugt wird.

Aus J. Amer. Chem. Soc. 70, 2297—98 (1948), ist es weiterhin bekannt, N-Benzoyl-d,l-allothreoninmethylester mittels Thionylchlorid in das entsprechende Oxazolin-hydrochlorid zu überführen, aus welchem durch Hydrolyse mit verdünnter Salzsäure d,l-Threonin erhalten wird. Hieran schließt sich die Feststellung, mit der beschriebenen Oxazolin-Inversion sei eine generell anwendbare Methode für die gegenseitige Umwandlung diastereomerer $\alpha,\beta$-Aminoalkohole mit hohen Ausbeuten gegeben. Bei den beschriebenen Verfahren dienen jeweils sekundäre N-Acylaminoalkohole als Ausgangsmaterial, wobei das am Stickstoffatom stehende Wasserstoffatom bei der Bildung des Oxazolinringes eliminiert wird.

Gemäß Chemical Abstracts 84, 43 593 x (1976), werden Derivate des Phenyl- und Phenoxyäthanolamins mit Halogenameisensäureestern umgesetzt und die erhaltenen N-Alkoxycarbonyl-Verbindungen mittels Thionylchlorid oder -bromid zu den entsprechenden Oxazolidinonen cyclisiert. Hieraus werden durch alkalische Hydrolyse oder Reduktion mittels Lithiumaluminiumhydrid die den Ausgangsstoffen entsprechenden Verbindungen mit umgekehrter sterischer Konfiguration erhalten.

H. K. Müller, Ann. 599, 211—221 (1956) gibt unter Bezugnahme auf die oben erläuterte Literaturstelle W. N. Nagai und S. Kanao an, daß die Umlagerung des N-Benzoyl-d,l-norephedrins mit günstigeren Ausbeuten gelingt, wenn man an Stelle der Schwefelsäure Thionylchlorid verwendet, und daß sich wegen der leichteren Verseifbarkeit des entstehenden Zwischenproduktes das N-Acetyl-d,l-norephedrin als Ausgangsmaterial für d,l-Pseudoephedrin noch besser eignet. Ausgehend von dem N-Acetylderivat wird auch ein unbeständiges Vorprodukt zum entsprechenden Oxazolin, das 2-Chlor-2,4-dimethyl-5-phenyl-oxazolidin-hydrochlorid, isoliert und außerdem die Struktur des Oxazolins durch Herstellung des isomeren d,l-threo-1-Phenyl-1-chlor-2-acetamido-propan-hydrochlorid mit deutlich verschiedenen Eigenschaften durch Umsetzung von d,l-Norephedrin mit Thionylchlorid und nachfolgende N-Acetylierung erhärtet.

Aus dem vorliegenden Stand der Technik ergibt sich, daß für die Konfigurations-Umkehrung von $\alpha$-Aminoalkoholen mit primärer Aminogruppe vor allem in der N-Acylierung, insbesondere N-Acetylierung, Überführung der N-Acylderivate in die entsprechenden Oxazoline mittels Thionylchlorid und, vorzugsweise alkalischen, Hydrolyse ein vorteilhaftes Verfahren zur Verfügung steht und weitere Verfahren ebenfalls in Betracht kommen.

Dagegen wurde die Konfigurations-Umkehrung von $\alpha$-Aminoalkohlen mit sekundärer Aminogruppe bisher nur durch N-Acylierung, Kochen des N-Acylderivates mit konz. Schwefelsäure im Acetanhydrid und Hydrolyse des Reaktionsproduktes unbekannter Konstitution mit verdünnter Schwefelsäure realisiert. Bei der Anwendung dieses Verfahrens auf das d-Pseudoephedrin wurde eine Ausbeute von nur 65% Inversionsprodukt erhalten, was bei einer monostereomeren Verbindung einer Ausbeute von lediglich ca. 30% Inversionsprodukt neben ca. 70% erneut aufzutrennendem Racemat entsprechen

würde. Zudem, und in erster Linie, erforderte die Umwandlung der N-Acylverbindung in das Oxazolin ziemlich energische Reaktionsbedingungen. Andererseits ist es z. B. aus Chemical Reviews 54, 614—685, besonders 632 und 633, (1954) und der dort zitierten Literatur bekannt, daß manche Aryl-alkyläther bereits beim Kochen mit Schwefelsäure in Eisessig gespalten werden. Ferner wurde gefunden, daß die analoge Anwendung des vorhin erwähnten, von Nagai und Kanao beschriebenen Verfahrens auf die Konfigurationsumkehr etwa im S(—)-1-(4-Benzyloxy-phenoxy)-3-isopropylamino-2-propanol zwecks Herstellung der entsprechenden R(+)-Verbindung jedoch nur zu einem, im Wesentlichen infolge Ätherspaltung stark verunreinigten Produkt führt. Ein solches Resultat legte den Schluß nahe, daß das bekannte Verfahren auf Ätherderivate des 3-Alkylamino-2-propanol nur zu unbefriedigenden Resultaten führen würde.

In Abetracht dessen konnte nicht erwartet werden, daß in optisch aktiven Verbindungen vom Typus der 1-Aryloxy-3-sekundäramino-2-propanole eine ähnliche Konfigurations-Umkehrung durchgeführt werden könnte. Die bei α-Primäramino-alkoholen unter milderen Reaktionsbedingungen mit gutem Erfolg durchführbare Reaktionsfolge der Umsetzung eines N-Acylderivats, z. B. der N-Acetylverbindung, z. B. mit Thionylchlorid zu einer Oxazolinverbindung als mittlerer Verfahrensstufe schien zur Anwendung bei 1-Aryloxy-3-sekundäramino-2-propanolen von vornherein nicht in Betracht zu kommen, weil die Bildung eines Oxazolins wegen des Fehlens eines Wasserstoffatoms, am Stickstoffatom eines N-acylierten Ausgangsstoffs nicht mehr möglich erschien.

Überraschenderweise wurde nun gefunden, daß man eine Umkehrung der Konfiguration in optisch aktiven Verbindungen der Formel I bewirken kann, indem man eine optisch aktive Verbindung der Formel

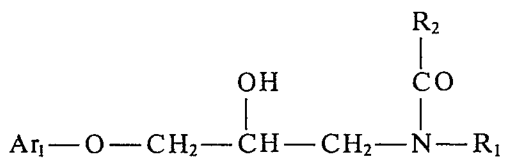

(II)

mit der R(+)- bzw. der S(—)-Konfiguration, worin $R_2$ einen gegebenenfalls substituierten mono- oder polycyclischen, carbo- oder heterocyclischen Rest, oder einen gegebenenfalls substituierten aliphatischen, cycloaliphatischen oder araliphatischen Kohlenwasserstoffrest darstellt, durch Behandeln mit einer starken, sauerstoffhaltigen anorganischen oder organischen Säure oder deren Halogeniden in eine optisch aktive Verbindung der Formel

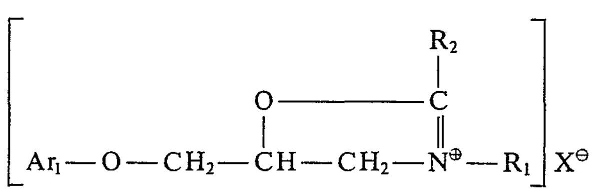

(III)

mit der R(—)- bzw. der S(+)-Konfiguration, worin $X^{\ominus}$ für das Anion einer starken, sauerstoffhaltigen anorganischen oder organischen Säure oder eines Halogenatoms steht, umwandelt, und die so erhaltene Verbindung der Formel III gegebenenfalls über die entsprechende freie Base als Zwischenstufe, zu einer Verbindung der Formel I mit einer Konfiguration, die derjenigen des verwendeten Ausgangsmaterials entgegengesetzt ist, hydrolysiert, und wenn erwünscht, eine freie Verbindung der Formel I in ein Salz oder ein erhaltenes Salz in die freie Verbindung überführt.

Als starke, sauerstoffhaltige anorganische oder organische Säuren kommen z. B. konzentrierte Schwefelsäure oder Phosphorsäure, Phosphorpentoxid oder eine starke organische Sulfonsäure, etwa eine aliphatische Sulfonsäure, z. B. Methansulfonsäure, oder eine aromatische Sulfonsäure, wie eine gegebenenfalls substituierte Phenylsulfonsäure, etwa 4-Methyl-, 4-Brom-, 4-Nitro- oder 2,4-Dinitrophenylsulfonsäure oder eine Naphthylsulfonsäure z. B. 1-Naphthylsulfonsäure, oder deren Halogenide, in erster Linie die Chloride oder Bromide wie Thionylchlorid, Thionylbromid, Sulfurylchlorid, Chlorsulfonsäure, Phosphortrichlorid, Phosphorpentachlorid, Phosphoroxychlorid oder Methansulfonylchlorid in Betracht. Ferner können auch den genannten Halogeniden entsprechende gemischte Ester, wie etwa ein Niederalkoxysulfonylhalogenid, z. B. Methoxy- oder Äthoxysulfonylchlorid oder Phosphorsäure-niederalkylesterhalogenide, z. B. Phosphorsäuredimethylester-chlorid verwendet werden.

Demnach bedeutet $X^{\ominus}$ in der Formel III das Anion insbesondere von Chlor oder Brom, der Schwefelsäure, Phosphorsäure oder einer starken organischen Sulfonsäure, z. B. einer der genannten.

Die cyclisierende Kondensation wird, sofern man eine der genannten Säuren als Kondensationsmittel einsetzt, üblicherweise in einem Temperaturbereich von etwa +50 bis +200°, bei Verwendung eines Halogenids von —10° bis +70°, vorzugsweise von +10 bis +50° durchgeführt.

Die Hydrolyse wird in saurem oder basischem Medium durchgeführt. Geeignete saure Mittel sind z. B. wäßrige Säuren, etwa wäßrige Mineralsäuren, z. B. wäßrige Salzsäure, Schwefelsäure oder

Phosphorsäure. Die saure Hydrolyse wird in einem Temperaturbereich von 0° bis +120°, zweckmäßigerweise bei +10 bis +50° durchgeführt. Als basische Medien sind z. B. wässerige Laugen, etwa die der Alkalien oder Erdalkalien wie Natriumhydroxid, oder Kaliumhydroxid, oder die Hydroxide des Calciums oder Magnesiums geeignet, wobei die genannten Reagentien vorteilhafterweise bei erhöhter Temperatur, etwa in einem Bereich von 50 bis 150° eingesetzt werden.

Die Hydrolyse kann auch stufenweise durchgeführt werden, indem man eine Verbindung der Formel III gegebenenfalls über die entsprechende freie Base als Zwischenstufe in wäßrigem Medium zu der entsprechenden N-Acylverbindung der Formel II mit umgekehrter sterischer Konfiguration wie die des jeweils verwendeten Ausgangsmaterials der Formel II hydrolysiert, und anschließend diese Verbindung zu einer der Formel I entsprechenden Verbindung hydrolysiert.

Das erfindungsgemäße Verfahren kann vorteilhafterweise auch so durchgeführt werden, daß ein in situ eingesetztes Ausgangsprodukt der Formel II, ohne dieses in reiner Form zu isolieren, im gleichen Reaktionsansatz zu der Verbindung der Formel III weiter verarbeitet und die erhaltene Verbindung der Formel III ohne weitere Reinigung der Hydrolyse unterworfen wird.

In einem Ausgangsmaterial der Formel II oder einem Zwischenprodukt der Formel III hat $R_2$ z. B. eine dem Rest $Ar_1$ oder dem Rest $R_1$ entsprechende Bedeutung und stellt insbesondere gegebenenfalls, z. B. wie angegeben, substituiertes Phenyl, Naphthyl oder Pyridyl, ferner Niederalkyl, wie Isopropyl oder tert.-Butyl, Phenylniederalkyl oder Phenyloxyniederalkyl mit gegebenenfalls am Verknüpfungskohlenstoffatom verzweigtem Niederalkyl, z. B. 1-Methyl-3-phenylpropyl bzw. 1-Methyl-3-phenoxypropyl dar.

Die Erfindung betrifft weiterhin eine spezielle Ausführungsform des erfindungsgemäßen Verfahrens, bei welchem man einen optisch aktiven Ausgangsstoff der Formel II, worin $Ar_1$ für Naphthyl, Niederalkyl-phenyl, Niederalkenyl-phenyl, Niederalkenyloxy-phenyl, Niederalkanoylamino-phenyl, Niederalkoxyniederalkyl-phenyl, 9,10-Äthano-9,10-dihydro-benzanthryl, Pyrazinyl, Pyrimidinyl, Indolyl, Benzofuryl, Cyano-pyridyl, Pyrryl-phenyl, Niederalkoxycarbonylaminoniederalkyl-pyridyl, N'-Cycloalkyl-ureido-phenyl, (Benzimidazol-2-on)-yl, Phenylniederalkoxyphenyl, Hydroxyphenyl, $R_1$ für Niederalkyl, Phenylniederalkyl, Phenoxyniederalkyl oder Pyridyloxyniederalkyl steht, und $R_2$ Niederalkyl, Phenylniederalkyl, Phenoxyniederalkyl oder Phenyl bedeutet, mit konzentrierter Phosphorsäure oder konzentrierter Schwefelsäure oder einem Halogenid solcher Säuren umsetzt und die so erhaltene Verbindung der Formel III, gegebenenfalls über die entsprechende freie Base als Zwischenstufe, zur entsprechenden Verbindung der Formel I mit entgegengesetzter Konfiguration hydrolysiert.

Die Erfindung betrifft weiterhin eine spezielle Ausführungsform des erfindungsgemäßen Verfahrens, bei welchem man einen optisch aktiven Ausgangsstoff der Formel II, worin $Ar_1$ für 1-Naphthyl, 3-Niederalkyl-phenyl, 2-Niederalkenyl-phenyl, 2-Niederalkenyloxy-phenyl, 4-Niederalkanoylamino-phenyl, 4-(2-Niederalkoxy-äthyl)-phenyl, 2-Pyrazinyl, 2-Pyrimidinyl, (1-Pyrryl)-phenyl, 4-Indolyl, 5-(2-Niederalkoxycarbonylamino-äthyl)-pyridyl, 3-Cyanopyridyl, 4-(Benzimidazol-2-on)-yl, 4-(N'-Cycloalkyl-ureido)-phenyl, Benzyloxy-phenyl, Hydroxyphenyl und $R_1$ für Niederalkyl, Phenylniederalkyl oder Phenoxyniederalkyl steht, und $R_2$ Niederalkyl, Phenylniederalkyl oder Phenyl bedeutet, mit konzentrierter Phosphorsäure oder konzentrierter Schwefelsäure oder einem Chlorid oder Bromid solcher Säuren umsetzt und die so erhaltene Verbindung der Formel III, gegebenenfalls über die entsprechende freie Base als Zwischenstufe zur entsprechenden Verbindung der Formel I mit entgegengesetzter Konfiguration hydrolysiert.

Die Erfindung betrifft weiterhin eine spezielle Ausführungsform des erfindungsgemäßen Verfahrens, bei welchem man einen optisch aktiven Ausgangsstoff der Formel II, in dem $Ar_1$ für 1-Naphthyl, 3-Methyl-phenyl, 2-Allyl-phenyl, 2-Allyloxy-phenyl, 4-Acetylamino-phenyl, 4-Indolyl, 4-(2-methoxyäthyl)-phenyl, 2-Pyrazinyl, 2-Pyrimidinyl, 2-(1-Pyrryl)-phenyl, 4-(N'-Cyclohexylureido)-phenyl, 4-Benzyloxy-phenyl oder 4-Hydroxy-phenyl $R_1$ für Niederalkyl oder Phenylniederalkyl und $R_2$ für Niederalkyl, Phenylniederalkyl oder Phenyl steht, mit konzentrierter Phosphorsäure oder konzentrierter Schwefelsäure oder einem Chlorid oder Bromid solcher Säuren umsetzt und die so erhaltene Verbindung der Formel III, gegebenenfalls über die entsprechende freie Base als Zwischenstufe, mittels wässeriger Mineralsäure oder wässeriger Alkalilauge zur entsprechenden Verbindung der Formel I mit entgegengesetzter Konfiguration hydrolysiert.

Die Erfindung betrifft weiterhin eine spezielle Ausführungsform des Verfahrens, bei welchem man einen optisch aktiven Ausgangsstoff der Formel II, in dem $Ar_1$ für 2-(1-Pyrryl)-phenyl, 1-Naphthyl, 2-Allyl-phenyl, 2-Allyloxy-phenyl, 4-(N'-Cyclohexylureido)-phenyl, 2-Pyrazinyl, 4-Benzyloxyphenyl oder 4-Hydroxyphenyl, $R_1$ für Niederalkyl und $R_2$ für Niederalkyl oder Phenyl steht, mit konzentrierter Schwefelsäure oder einem Chlorid davon, oder mit Thionylchlorid umsetzt und die so erhaltene Verbindung der Formel III, gegebenenfalls über die entsprechende freie Base als Zwischenstufe, mittels wässeriger Salzsäure oder Schwefelsäure oder mittels wässeriger Alkalilauge zur entsprechenden Verbindung der Formel I mit entgegengesetzter Konfiguration hydrolysiert.

Die Erfindung betrifft insbesondere die in den Beispielen beschriebenen Verfahren.

Die Erfindung betrifft ferner optisch aktive Verbindungen der Formel III mit der R(−)- bzw. der S(+)-Konfiguration, worin $Ar_1$, $R_1$, $R_2$ und X obige Bedeutungen haben, oder solche Verbindungen als freie Basen.

Die Erfindung betrifft weiterhin optisch aktive Verbindungen der Formel III, worin X Halogen oder den Rest der Schwefelsäure oder Phosphorsäure bedeutet, $Ar_1$ für Naphthyl, Niederalkyl-phenyl, Niederalkenyl-phenyl, Niederalkenyloxy-phenyl, Niederalkanoylamino-phenyl, Niederalkoxyniederalkyl-phenyl, 9,10-Äthano-9,10-dihydro-benzanthryl, Pyrazinyl, Pyrimidinyl, Indolyl, Benzofuryl, Cyano-pyridyl, Pyrryl-phenyl, Niederalkoxycarbonylaminoniederalkyl-pyridyl, N'-Cycloalkyl-ureido-phenyl, (Benzimidazol-2-on)-yl, Phenylniederalkoxy-phenyl, Hydroxyphenyl, $R_1$ für Niederalkyl, Phenylniederalkyl, Phenoxyniederalkyl oder Pyridyloxyniederalkyl steht, und $R_2$ Niederalkyl, Phenylniederalkyl, Phenoxyniederalkyl oder Phenyl bedeutet, oder solche Verbindungen als freie Basen.

Die Erfindung betrifft weiterhin optisch aktive Verbindungen der Formel III, worin X Chlor oder Brom oder den Rest der Schwefelsäure oder Phosphorsäure bedeutet, $Ar_1$ für 1-Naphthyl, 3-Niederalkyl-phenyl, 2-Niederalkenyl-phenyl, 2-Niederalkenyloxy-phenyl, 4-Niederalkanoylamino-phenyl, 4-(2-Niederalkoxy-äthyl)-phenyl, 2-Pyrazinyl, 2-Pyrimidinyl, (1-Pyrryl)-phenyl, 4-Indolyl, 5-(2-Niederalkoxycarbonylamino-äthyl)-pyridyl, 3-Cyano-pyridyl, 4-(Benzimidazol-2-on)-yl, 4-(N'-Cycloalkyl-ureido)-phenyl, Benzyloxy-phenyl, Hydroxyphenyl und $R_1$ für Niederalkyl, Phenylniederalkyl oder Phenoxy-niederalkyl steht, und $R_2$ Niederalkyl, Phenylniederalkyl oder Phenyl bedeutet, oder solche Verbindungen als freie Basen.

Die Erfindung betrifft weiterhin optisch aktive Verbindungen der Formel III, worin X Chlor oder Brom oder den Rest der Schwefelsäure oder Phosphorsäure bedeutet, $Ar_1$ für 1-Naphthyl, 3-Methyl-phenyl, 2-Allyl-phenyl, 2-Allyloxy-phenyl, 4-Acetylamino-phenyl, 4-Indolyl, 4-(2-Methoxyäthyl)-phenyl, 2-Pyrazinyl, 2-Pyrimidinyl, 2-(1-Pyrryl)-phenyl, 4-(N'-Cyclohexylureido)-phenyl, 4-Benzyloxy-phenyl oder 4-Hydroxy-phenyl, $R_1$ für Niederalkyl oder Phenylniederalkyl und $R_2$ für Niederalkyl, Phenylniederalkyl oder Phenyl steht, oder solche Verbindungen als freie Basen.

Die Erfindung betrifft weiterhin optisch aktive Verbindungen der Formel III, worin X Chlor oder den Rest der Schwefelsäure darstellt, $Ar_1$ für 2-(1-Pyrryl)-phenyl, 1-Naphthyl, 2-Allyl-phenyl, 2-Allyloxy-phenyl, 4-(N'-Cyclohexylureido)-phenyl, 2-Pyrazinyl, 4-Benzyloxyphenyl oder 4-Hydroxyphenyl, $R_1$ für Niederalkyl und $R_2$ für Niederalkyl oder Phenyl steht oder solche Verbindungen als freie Basen.

Die Erfindung betrifft speziell die in den Beispielen beschriebenen Verbindungen der Formel III.

Je nach den Verfahrensbedingungen und Ausgangsstoffen erhält man die optisch aktiven Verbindungen der Formel I in freier Form oder in der ebenfalls von der Erfindung umfaßten Form ihrer Salze, wobei die neuen Verbindungen oder Salze davon auch als Hemi-, Mono-, Sesqui- oder Polyhydrate davon vorliegen können. Säureadditionssalze der neuen Verbindungen können in an sich bekannter Weise, z. B. durch Behandeln mit basischen Mitteln, wie Alkalimetallhydroxiden, -carbonaten oder -hydrogencarbonaten oder Ionenaustauschern, in die freien Verbindungen übergeführt werden. Andererseits können erhaltene freie Basen mit organischen oder anorganischen Säuren, z. B. mit den genannten Säuren, Säureadditionssalze bilden, wobei zu deren Herstellung insbesondere solche Säuren verwendet werden, die sich zur Bildung von pharmazeutisch annehmbaren Salzen eignen.

Diese oder andere Salze, insbesondere Säureadditionssalze der neuen Verbindungen, wie z. B. Pikrate oder Perchlorate, können auch zur Reinigung der erhaltenen freien Basen dienen, indem man die freien Basen in Salze überführt, diese abtrennt und reinigt, und aus den Salzen wiederum die Basen freisetzt.

Salze von optisch aktiven Verbindungen der Formel I stellen Säureadditionssalze, und insbesondere pharmazeutisch annehmbare, nicht-toxische Säureadditionssalze mit geeigneten anorganischen Säuren, wie Chlorwasserstoffsäure, Bromwasserstoffsäure, Schwefelsäure oder Phosphorsäure, oder mit geeigneten organischen, wie aliphatischen cycloaliphatischen, aromatischen, araliphatischen oder heterocyclischen Carbon- oder Sulfonsäuren, wie Ameisensäure, Essigsäure, Propionsäure, Bernsteinsäure, Glykolsäure, Milchsäure, Äpfelsäure, Weinsäure, Zitronensäure, Ascorbinsäure, Maleinsäure, Fumarsäure, Brenztraubensäure, Benzoesäure, Anthranilsäure, 4-Hydroxybenzoesäure, Salicylsäure, Phenylessigsäure, Embonsäure, Methansulfonsäure, Äthansulfonsäure, Hydroxyäthansulfonsäure, Äthylensulfonsäure, 4-Chlorbenzolsulfonsäure, Toluolsulfonsäure, Naphthalinsulfonsäure, Sulfanilsäure oder Cyclohexylaminsulfonsäure, sowie Ascorbinsäure dar. Infolge der engen Beziehungen zwischen den neuen Verbindungen in freier Form und in Form ihrer Salze sind unter den freien Verbindungen und unter den Salzen sinn- und zweckmäßig gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder bei denen man einen Ausgangsstoff unter den Reaktionsbedingungen bildet, oder bei denen eine Reaktionskomponente gegebenenfalls in Form ihrer Salze vorliegt.

Zweckmäßig verwendet man für die Durchführung der erfindungsgemäßen Reaktionen solche Ausgangsstoffe, die zu den eingangs besonders erwähnten Gruppen von Endstoffen und besonders zu den speziell beschriebenen oder hervorgehobenen Endstoffen führen.

Die Ausgangsstoffe sind bekannt oder können, falls sie neu sind, nach an sich bekannten Methoden, z. B. analog wie in den Beispielen beschrieben, erhalten werden. So kann man beispielsweise eine Verbindung der Formel (I) mit einer Carbonsäure der Formel $R_2$—COOH (IIa), worin $R_2$ die angegebene

Bedeutung hat, oder einem reaktionsfähigen Derivat davon, etwa einem geeignet reaktionsfähigen Ester, z. B. einem Niederalkyl- wie Methylester oder einem aromatischen Ester, wie Phenyl, 4-Nitrophenyl- oder 2,4-Dinitrophenylester, oder einem Halogenid, wie Chlorid oder Bromid, oder einem Anhydrid, wie einem symmetrischen oder gemischten Anhydrid, in an sich bekannter Weise zu der entsprechend acylierten Verbindung der Formel (II) umsetzen.

Die folgenden Beispiele dienen zur Illustration der Erfindung; Temperaturen werden in Celsiusgraden angegeben.

## Beispiel 1

Zu einer Lösung von 26,2 g R(+)-1-(4-Benzyloxyphenoxy)-3-isopropylamino-2-propanol in 78 ml Dimethylformamid werden bei 10° 18 g Acetanhydrid getropft. Die Lösung wird 2 Stunden bei 20° gerührt, dann auf 300 ml Wasser gegossen und mit 160 ml Toluol extrahiert. Die Toluollösung wird mit Natriumhydrogencarbonatlösung gewaschen, über Natriumsulfat getrocknet und in Vakuum eingedampft, wonach man das rohe R(+)-1-(4-Benzoyloxyphenoxy)-3-N-isopropyl-N-acetylamino-2-propanol erhält.

Dieses wird in 40 ml Methylenchlorid gelöst und bei 5—10° innerhalb 45 Minuten zu einer Lösung von 6,2 ml Thionylchlorid in 18 ml Methylenchlorid zugetropft. Die Lösung wird 2 Stunden bei 20°, dann 1 Stunde bei 35° gerührt und anschließend in Vakuum zur Trockne eingedampft.

Man erhält 32 g (93%) rohres S(—)-1-Isopropyl-2-methyl-5-(4-benzyloxy-phenoxymethyl)-oxazolinium-chlorid als dickes, bald kristallisierendes Öl. Die hygroskopischen Kristalle enthalten 1 Mol Kristall-Chlorwasserstoff; nach dem Umkristallisieren aus einem Gemisch von Methylenchlorid-Essigsäureäthylester schmilzt die Verbindung bei 78—82°.

32 g des rohen S(—)-1-Isopropyl-2-methyl-5-(4-benzyloxy-phenoxymethyl)-oxazoliniumchlorids werden mit 25 ml Wasser, 100 ml Äthanol und 16 g Natriumhydroxid versetzt und das Gemisch 1 Stunde am Rückfluß gekocht, wonach das Reaktionsgemisch auf 300 ml Wasser gegossen und auf 0° gekühlt wird. Die ausgefallenen Kristalle werden abgesaugt und getrocknet, wonach man 23,6 g (90% d. Th.) S(—)-1-(4-Benzyloxyphenoxy)-3-isopropylamino-2-propanol erhält.

$[\alpha]_D^{20}$ Ausgangsprodukt: ≈ +25° (1%ige Lösung in 1-n Schwefelsäure)
$[\alpha]_D^{20}$ Endprodukt: ≈ —22° (1%ige Lösung in 1-n Schwefelsäure)
Optische Ausbeute: 87%

Das R(+)-1-(4-Benzyloxyphenoxy)-3-isopropylamino-2-propanol kann beispielsweise wie folgt erhalten werden:

a) 4,5 g (±)-1-(4-Hydroxyphenoxy)-2-hydroxy-3-(isopropylamino)-propan werden in 30 ml Methanol gelöst und mit einer Lösung von 8,1 g (—)-Di-O,O'-p-toluoyl-L-Weinsäure in 25 ml Methanol und 25 ml Wasser versetzt. Dann wird die Lösung bei Raumtemperatur im Wasserstrahlvakuum bis zum Einsetzen einer leichten Trübung eingeengt und anschließend zur Kristallisation 15 Stunden bei 0° stehen gelassen. Die ausgefallenen Kristalle werden abgesaugt und anschließend zweimal aus Methanol-Wasser 1 : 1 umkristallisiert. Man erhält das R(+)-1-(4-Hydroxyphenoxy)-2-hydroxy-3-isopropylamino-propan-(—)-di-O,O'-p-toluoyl-L-hydrogentartat; Smp. 134—137°.

Zur Isolierung der freien enantiomeren Base wird das Salz in 2-n-Salzsäure aufgenommen und mit Essigsäureäthylester ausgeschüttelt. Aus diesem Extrakt kann die (—)-Di-O,O'-p-toluoyl-D-Weinsäure regeneriert werden. Die salzsaure Lösung wird mit konzentriertem Ammoniak alkalisch gestellt, mit Natriumchlorid gesättigt und mit Essigsäureäthylester ausgeschüttelt. Die organische Phase wird mit Sole gewaschen, über Natriumsulfat getrocknet und im Wasserstrahlvakuum eingedampft. Der Rückstand kristallisiert aus Essigsäureäthylester, wonach man reines R(+)-1-(4-Hydroxyphenoxy)-3-isopropylamino)-2-propanol vom Smp. 129—130° erhält.

$[\alpha]_D^{20}$: + 0,9±0,5° (1%ige Lösung in Methanol).
$[\alpha]_D^{20}$: +20,5±0,5° (1%ige Lösung in 1-n Schwefelsäure).

b) Eine Lösung von 52 g R(+)-1-(4-Hydroxyphenoxy)-3-isopropylamino-2-propanol-hydrochlorid in Dimethylformamid wird bei Raumtemperatur mit 42 g einer 50%igen Lösung von Natriumhydroxid in Wasser versetzt. Zu diesem Gemisch werden bei 30° in 15 Minuten 33 g Benzylbromid zugetropft und 1 Stunde bei Raumtemperatur gerührt. Das Reaktionsgemisch wird in 600 ml auf 0° gekühltes Wasser eingetragen, 1 Stunde bei 0° gerührt, die ausgefallenen Kristalle abgesaugt und getrocknet, wonach man R(+)-1-(4-Benzyloxyphenoxy)-3-isopropylamino-2-propanol vom Smp. 91—93° erhält.

Beispiel 2

In 25 ml Acetanhydrid werden unter Rühren portionenweise 6,5 g S(−)-1-(4-Hydroxyphenoxy)-3-isopropylamino-2-propanol eingetragen, wonach unter Wärmeentwicklung eine klare Lösung entsteht. Diese versetzt man mit einer Lösung von 3 g Schwefelsäure (96%) in 10 ml Acetanhydrid und erhitzt das Gemisch während 2 Stunden unter Rückfluß. Anschließend wird die erhaltene Lösung im Vakuum eingeengt. Der Rückstand stellt rohes R(+)-1-Isopropyl-2-methyl-5-(4-hydroxyphenoxymethyl)-oxazolinium-sulfat dar, welches in 50 ml 1-n. Schwefelsäure aufgenommen und 2 Stunden unter Rückfluß erhitzt wird. Dann werden 100 g Eis zugegeben und mit Ammoniak auf pH 9 eingestellt. Die Lösung wird 4mal mit je 100 ml Essigsäureäthylester extrahiert, die vereinigten organischen Phasen über Natriumsulfat getrocknet und zur Trockne verdampft. Man erhält 5,5 g, 85% d. Th. R(+)-1-(4-Hydroxyphenoxy)-3-isopropylamino-2-propanol.

$[\alpha]_D^{20}$ Ausgangsmaterial: −20,5° (1%ige Lösung in 1-n Schwefelsäure)
$[\alpha]_D^{20}$ Endprodukt: +17,3° (1%ige Lösung in 1-n Schwefelsäure)
Optische Ausbeute: 85%.

Beispiel 3

In 25 ml Acetanhydrid werden unter Rühren portionsweise 6,5 g S(−)-1-(4-Benzyloxyphenoxy)-3-isopropylamino-2-propanol eingetragen, wonach unter Wärmeentwicklung eine klare Lösung entsteht. Diese versetzt man mit einer Lösung von 3 g Schwefelsäure (96%) in 10 ml Acetanhydrid und erhitzt das Gemisch während 2 Stunden unter Rückfluß. Anschließend wird die erhaltene Lösung im Vakuum eingeengt, der Rückstand in 50 ml 1-n Schwefelsäure aufgenommen und 2 Stunden unter Rückfluß erhitzt. Dann werden 100 g Eis zugegeben und mit Ammoniak auf pH 9 eingestellt. Die Lösung wird 4mal mit je 100 ml Essigsäureäthylester extrahiert, die vereinigten organischen Phasen über Natriumsulfat getrocknet und dann zur Trockne verdampft. Man erhält 3,5 g eines Rückstandes, welcher stark verunreinigtes R(+)-1-(4-Hydroxyphenoxy)-3-isopropylamino-2-propanol darstellt.

Beispiel 4

0,9 g D(+)-1-(o-Allyloxyphenoxy)-3-isopropylamino-2-propanol werden in 5 ml Dimethylformamid gelöst und hierzu bei 10° 0,34 ml Acetanhydrid zugetropft. Die Lösung wird 2 Stunden bei 20° gerührt, auf 25 ml Wasser gegossen und zweimal mit je 30 ml Toluol ausgezogen. Die Toluollösung wird nach dem Trocknen über Magnesiumsulfat in Vakuum zur Trockne eingedampft, wonach man das rohe D(+)-1-(o-Allyloxyphenoxy)-3-N-isopropyl-N-acetylamino-2-propanol erhält. Dieses wird in 5 ml Methylenchlorid gelöst und bei 5—10° innerhalb 45 Minuten zu einer Lösung von 0,6 ml Thionylchlorid in 5 ml Methylenchlorid getropft, anschließend 2 Stunden bei 20°, dann 1 Stunde bei 35° gerührt und anschließend in Vakuum zur Trockne eingedampft. Der Rückstand stellt das rohe L(−)-1-Isopropyl-2-methyl-5-(o-allyloxy-phenoxymethyl)-oxazoliniumchlorid dar. Die Lösung dieser Verbindung in einem Gemisch von 1 ml Wasser und 10 ml Äthanol wird mit 1,0 g Natriumhydroxid versetzt und das Gemisch 1 Stunde unter Rückfluß gekocht, dann auf 25 ml Wasser gegossen, die Lösung zweimal mit je 25 ml Toluol ausgezogen und die vereinigten organischen Phasen nach dem Trocknen über Magnesiumsulfat im Vakuum zur Trockne verdampft. Der Rückstand wird in 2,5 ml Cyclohexanol gelöst und die Lösung abgekühlt; die dabei ausgefallenen Kristalle werden abgesaugt, wonach man 0,7 g (78% d. Th.) L(−)-1-(o-Allyloxyphenoxy)-3-isopropylamino-2-propanol erhält.

$[\alpha]_D$ Ausgangsprodukt: +13,5° ±0,5 (1%ige Lösung in 1-n Schwefelsäure)
$[\alpha]_D$ Endprodukt: −12,7° ±0,5 (1%ige Lösung in 1-n Schwefelsäure)
Optische Ausbeute: 94%.

Das als Ausgangsmaterial verwendete D(+)-1-(o-Allyloxyphenoxy)-3-isopropylamino-2-propanol kann wie folgt hergestellt werden:

265 g (1 Mol) racemisches 1-(o-Allyloxyphenoxy-3-isopropylamino-2-propanol und 140 g (0,95 Mol) L(+)-Glutaminsäure ($[\alpha]_{546}^{20}$ = +36±2°, 5%ige Lösung in 5-n wässeriger Salzsäure) werden im Mörser gründlich gemischt und die Mischung in 470 ml auf 70° erwärmten Wasser gelöst. Die warme Lösung wird klar filtriert, das farblose Filtrat mit 2300 ml 96%igem Äthanol versetzt und die Lösung 24 Stunden bei 50° stehen gelassen. Der ausgefallene kristalline Niederschlag wird abgesaugt, mit 200 ml Äthanol gewaschen und an der Luft getrocknet. Man erhält 220 g rohes D(+)-1-(o-Allyloxyphenoxy)-3-isopropylamino-2-propanol. L(+)-glutaminat vom Smp. 172—173°; dieses wird in 100 ml Wasser bei 70° gelöst und die Lösung mit 800 ml Methanol versetzt. Nach Stehen während 15 Stunden bei 5° wird der ausgefallene Niederschlag abgesaugt, wonach man 210 g des enantiomeren Salzes erhält, Smp. 176—178°. Eine weitere Umkristallisation unter den gleichen Bedingungen liefert 125 g des reinen

D(+)-1-(o-Allyloxyphenoxy)-3-isopropylamino-2-propanol. L(+)-glutaminat, welches nach dem Trocknen bei 60°/0,03 mm Hg während 8 Stunden folgende Daten zeigt: Smp. 179—181°;

$[\alpha]_{313}$: +34±1° (als 1-ige Lösung in Wasser)
$[\alpha]_D$: +7,2° (als 1%ige Lösung in Wasser).

Elementaranalyse berechnet für $C_{15}H_{23}NO_3 \cdot C_5H_9NO_4$

C (berechnet in %): 58,2, H (berechnet): 7,8,N (berechnet): 6,8
C (gefunden):58,2, H (gefunden):7,9, N (gefunden):6,7

Beim Versetzen der wässerigen Lösung von D(+)-1-(o-Allyloxyphenoxy)-3-isopropylamino-2-propanol. L(+)-glutaminat mit wässerigem Ammoniak bis zur alkalischen Reaktion, Extrahieren mit Methylenchlorid, Abdampfen desselben bis zur Trockne und Umkristallisieren des Rückstandes aus Äther und Hexan erhält man die freie Base vom Smp. 55—56°. Das Hydrochlorid hat folgende Eigenschaften:

Smp. 72—74°; $[\alpha]_D^{20}$: +19±1° (1%ige Lösung in Äthanol).

Aus den vereinigten Mutterlaugen wird durch Versetzen mit wässerigem Ammoniak bis zur alkalischen Reaktion, Extrahieren mit Methylenchlorid und Abdampfen desselben ein Produkt erhalten, in welchem der L(−)-Antipode in angereicherter Form vorliegt. Aus diesem Produkt kann durch analoge Anwendung des beschriebenen Verfahrens unter Verwendung von D(−)-Glutaminsäure das entsprechende L(−)-1-(o-Allyloxyphenoxy)-3-isopropylamino-2-propanol erhalten werden, dessen Hydrochlorid folgende Eigenschaften besitzt:

Smp. 73—75°; $[\alpha]_D^{20}$: −19° ±1° (1%ige Lösung in Äthanol).

## Beispiel 5

0,9 g (−)-1-(1-Naphthyloxy)-3-isopropylamino-2-propanol-hydrochlorid werden in 10 ml Wasser gelöst, mit 4 ml 1-n Natronlauge versetzt und das Gemisch dreimal mit je 25 ml Essigsäureäthylester ausgezogen. Die organische Lösung wird nach dem Trocknen über Magnesiumsulfat in Vakuum eingedampft, die als Rückstand erhaltene freie Base in 5 ml Dimethylformamid gelöst und zu dieser Lösung bei 10° 0,34 ml Acetanhydrid zugetropft. Die Lösung wird 2 Stunden bei 20° gerührt, auf 25 ml Wasser gegossen und zweimal mit je 30 ml Toluol ausgezogen. Die Toluollösung wird nach Trocknen über Magnesiumsulfat in Vakuum zur Trockne eingedampft, wonach man rohes (−)-1-(1-Naphthyloxy)-3-N-isopropyl-N-acetylamino-2-propanol erhält. Dieses wird in 5 ml Methylenchlorid gelöst und bei 5—10° innerhalb 45 Minuten zu einer Lösung von 0,6 ml Thionylchlorid in 5 ml Methylenchlorid getropft, anschließend 2 Stunden bei 20°, dann 1 Stunde bei 35° gerüht und anschließend in Vakuum zur Trockne eingedampft. Der ölige Rückstand stellt das rohe (+)-1-Isopropyl-2-methyl-5-[1-naphthyloxymethyl)-oxazoliniumchlorid dar. Die Lösung dieser Verbindung in 1 ml Wasser und 10 ml Äthanol wird mit 1 g Natriumhydroxid versetzt und das Gemisch 1 Stunde unter Rückfluß gekocht. Danach wird das Reaktionsgemisch auf 25 ml Wasser gegossen, zweimal mit je 25 ml Toluol ausgezogen und die Toluolschichten nach Trocknen über Magnesiumsulfat in Vakuum zur Trockne eingedampft.

Der Rückstand wird in 5 ml Isopropanol gelöst und mit einer Lösung von Salzsäuregas in Isopropanol gegen Methylrot neutralisiert. Die dabei ausgefallenen Kristalle werden abgesaugt, wonach 0,7 g (78% d. Th.) des (+)-1-(1-Naphthyloxy)-3-isopropylamino-2-propanol-hydrochlorid erhalten werden.

| | | |
|---|---|---|
| $[\alpha]_D$ Ausgangsprodukt: | −13,2° ±0,5 | (1%ige Lösung in Wasser, 20°) |
| $[\alpha]_D$ Endprodukt: | +13,7° ±0,5 | (1%ige Lösung in Wasser, 20°) |
| Optische Ausbeute: | 100%. | |

Das als Ausgangsmaterial verwendete (−)-1-(1-Naphthyloxy)-3-isopropylamino-2-propanol-1-hydrochlorid kann analog dem in Beispiel 4 beschriebenen Verfahren der Racemattrennung erhalten werden. Diese Ausgangsmaterialien zeigen folgende Eigenschaften:

(+)-1-(1-Naphthyloxy)-3-isopropylamino-2-propanol-hydrochlorid: Smp. 192—193°;
$[\alpha]_D^{20}$: +25±1° (1%ige Lösung in Äthanol),
(−)-1-(1-Naphthyloxy)-3-isopropylamino-2-propanol-hydrochlorid: Smp. 192—193°;
$[\alpha]_D^{20}$: −25±1° (1%ige Lösung in Äthanol).

**Patentansprüche für die Vertragsstaaten BE, CH, DE, FR, GB, T, LU, NL, SE**

1. Verfahren zur Umkehrung der Konfiguration in optisch aktiven Verbindungen der Formel

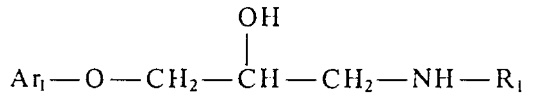

(I)

worin $Ar_1$ für einen gegebenenfalls substituierten mono- oder polycyclischen, carbo- oder heterocyclischen Rest steht, der mindestens einen Ring mit aromatischem Charakter aufweist und der über ein Ringkohlenstoffatom, vorzugsweise des Rings mit aromatischem Charakter, mit dem Sauerstoffatom verbunden ist, $R_1$ einen gegebenenfalls substituierten aliphatischen, cycloaliphatischen oder araliphatischen Kohlenwasserstoffrest bedeutet, oder deren Salzen, dadurch gekennzeichnet, daß man eine optisch aktive Verbindung der Formel

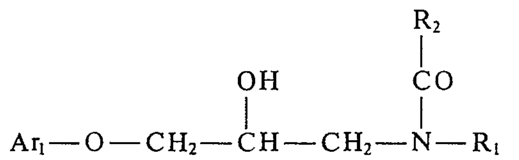

(II)

mit der R(+)- bzw. der S(−)-Konfiguration, worin $R_2$ einen gegebenenfalls substituierten mono- oder polycyclischen, carbo- oder heterocyclischen Rest oder einen gegebenenfalls substituierten aliphatischen, cycloaliphatischen oder araliphatischen Kohlenwasserstoffrest darstellt, durch Behandeln mit einer starken, sauerstoffhaltigen anorganischen oder organischen Säure oder deren Halogeniden in eine optisch aktive Verbindung der Formel

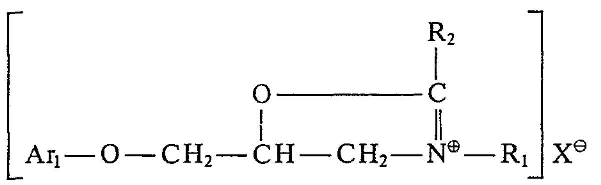

(III)

mit der R(−)- bzw. der S(+)-Konfiguration, worin $X^{\ominus}$ für das Anion einer starken, sauerstoffhaltigen anorganischen oder organischen Säure oder eines Halogenatoms steht, umwandelt, und die so erhaltene Verbindung der Formel III, gegebenenfalls über die entsprechende freie Base als Zwischenstufe, zu einer Verbindung der Formel I mit einer Konfiguration, die derjenigen des verwendeten Ausgangsmaterials entgegengesetzt ist, hydrolysiert, und wenn erwünscht, eine freie Verbindung der Formel I in ein Salz oder ein erhaltenes Salz in die freie Verbindung überführt.

2. Verfahren nach Anspruch 1, dadurch gekennzeichnet, daß man als starke, sauerstoffhaltige anorganische oder organische Säure konzentrierte Schwefelsäure, konzentrierte Phosphorsäure, Phosphorpentoxid oder eine starke organische Sulfonsäure verwendet.

3. Verfahren nach Anspruch 1, dadurch gekennzeichnet, daß man als Halogenid einer starken, sauerstoffhaltigen anorganischen oder organischen Säure Thionylchlorid, Thionylbromid, Sulfurylchlorid, Chlorsulfonsäure, Methansulfonylchlorid, Phosphortrichlorid, Phosphoroxychlorid oder Phosphorpentachlorid verwendet.

4. Verfahren nach Anspruch 1–2, dadurch gekennzeichnet, daß man bei Verwendung einer starken, sauerstoffhaltigen anorganischen oder organischen Säure in einem Temperaturbereich von +50 bis +200° arbeitet.

5. Verfahren nach Anspruch 1 oder 3, dadurch gekennzeichnet, daß man bei Verwendung eines Halogenids einer starken, sauerstoffhaltigen anorganischen oder organischen Säure in einem Temperaturbereich von −10 bis +70° arbeitet.

6. Verfahren nach Anspruch 1, dadurch gekennzeichnet, daß man die Hydrolyse in einem sauren oder basischen Medium durchführt.

7. Verfahren nach einem der Ansprüche 1–6, dadurch gekennzeichnet, daß man einen optisch aktiven Ausgangsstoff der Formel II, worin $Ar_1$ für Naphthyl, Niederalkylphenyl, Niederalkenyl-phenyl, Niederalkenyloxy-phenyl, Niederalkanoylamino-phenyl, Niederalkoxyniederalkyl-phenyl, 9,10-Äthano-9,10-dihydro-benzanthryl, Pyrazinyl, Pyrimidinyl, Indolyl, Benzofuryl, Cyano-pyridyl, Pyrryl-phenyl, Niederalkoxycarbonylaminoniederalkyl-pyridyl, N'-Cycloalkylureido-phenyl, (Benzimidazol-2-on)-yl, Phenylniederalkoxyphenyl oder Hydroxyphenyl und $R_1$ für Niederalkyl, Phenylniederalkyl, Phenoxyniederalkyl oder Pyridyloxyniederalkyl steht und $R_2$ Niederalkyl, Phenylniederalkyl, Phenoxyniederalkyl oder Phenyl bedeutet, mit konzentrierter Phosphorsäure oder konzentrierter Schwefelsäure oder einem Halogenid solcher Säuren umsetzt und die so erhaltene Verbindung der Formel III, gegebenenfalls über die entsprechende freie Base als Zwischenstufe, zur entsprechenden

Verbindung der Formel I mit entgegengesetzter Konfiguration hydrolysiert.

8. Verfahren nach einem der Ansprüche 1—6, dadurch gekennzeichnet, daß man einen optisch aktiven Ausgangsstoff der Formel II, worin $Ar_1$ für 1-Naphthyl, 3-Niederalkyl-phenyl, 2-Niederalkenyl-phenyl, 2-Niederalkenyloxyphenyl, 4-Niederalkanoylamino-phenyl, 4-(2-Niederalkoxyäthyl)-phenyl, 2-Pyrazinyl, 2-Pyrimidinyl, (1-Pyrryl)-phenyl, 4-Indolyl, 5-(2-Niederalkoxycarbonylamino-äthyl)-pyridyl, 3-Cyano-pyridyl, 4-(Benzimidazol-2-on)-yl, 4-(N'-Cycloalkyl-ureido)-phenyl, Benzyloxy-phenyl oder Hydroxyphenyl und $R_1$ für Niederalkyl, Phenylniederalkyl oder Phenoxyniederalkyl steht, und $R_2$ Niederalkyl, Phenylniederalkyl oder Phenyl bedeutet, mit konzentrierter Phosphorsäure oder konzentrierter Schwefelsäure oder einem Chlorid oder Bromid solcher Säuren umsetzt, und die so erhaltene Verbindung der Formel III, gegebenenfalls über die entsprechende freie Base als Zwischenstufe, zur entsprechenden Verbindung der Formel I mit entgegengesetzter Konfiguration hydrolysiert.

9. Verfahren nach einem der Ansprüche 1—6, dadurch gekennzeichnet, daß man einen optisch aktiven Ausgangsstoff der Formel II, in dem $Ar_1$ für 1-Naphthyl, 3-Methyl-phenyl, 2-Allyl-phenyl, 2-Allyloxy-phenyl, 4-Acetylamino-phenyl, 4-Indolyl, 4-(2-methoxyäthyl)-phenyl, 2-Pyrazinyl, 2-Pyrimidinyl, 2-(1-Pyrryl)-phenyl, 4-(N'-Cyclohexyl-ureido)-phenyl, 4-Benzyloxy-phenyl oder 4-Hydroxy-phenyl, $R_1$ für Niederalkyl oder Phenylniederalkyl und $R_2$ für Niederalkyl, Phenylniederalkyl oder Phenyl steht, mit konzentrierter Phosphorsäure oder konzentrierter Schwefelsäure oder einem Chlorid oder Bromid solcher Säuren umsetzt und die so erhaltene Verbindung der Formel III, gegebenenfalls über die entsprechende freie Base als Zwischenstufe, mittels wässeriger Mineralsäure oder wässeriger Alkalilauge zur entsprechenden Verbindung der Formel I mit entgegengesetzter Konfiguration hydrolysiert.

10. Verfahren nach einem der Ansprüche 1—6, dadurch gekennzeichnet, daß man einen optisch aktiven Ausgangsstoff der Formel II, worin $Ar_1$ für 2-(1-Pyrryl)-phenyl, 1-Naphthyl, 2-Allyl-phenyl, 2-Allyloxy-phenyl, 4-(N'-Cyclohexylureido)-phenyl, 2-Pyrazinyl, 4-Benzyloxy-phenyl oder 4-Hydroxy-phenyl, $R_1$ für Niederalkyl und $R_2$ für Niederalkyl oder Phenyl steht, mit konzentrierter Schwefelsäure oder einem Chlorid davon, oder mit Thionylchlorid umsetzt und die so erhaltene Verbindung der Formel III, gegebenenfalls über die entsprechende freie Base als Zwischenstufe, mittels wässeriger Salzsäure oder Schwefelsäure oder mittels wässeriger Alkalilauge zur entsprechenden Verbindung der Formel I mit entgegengesetzter Konfiguration hydrolysiert.

11. Optisch aktive Verbindungen der Formel

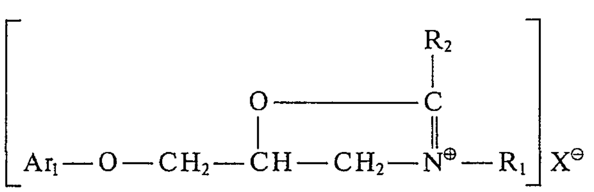

(III)

mit der R(—)- bzw. der S(+)-Konfiguration, worin $X^{\ominus}$ für das Anion einer starken, sauerstoffhaltigen anorganischen oder organischen Säure oder eines Halogenatoms steht und $Ar_1$, $R_1$ und $R_2$ die im Anspruch 1 angegebene Bedeutung haben, oder solche Verbindungen als freie Basen.

12. Optisch aktive Verbindungen der Formel III gemäß Anspruch 11, worin X Halogen oder den Rest der Schwefelsäure oder Phosphorsäure bedeutet, $Ar_1$ für Naphthyl, Niederalkyl-phenyl, Niederalkenylphenyl, Niederalkenyloxy-phenyl, Niederalkanoylamino-phenyl, Niederalkoxyniederalkyl-phenyl, 9,10-Äthano-9,10-dihydro-benz-anthryl, Pyrazinyl, Pyrimidinyl, Indolyl, Benzofuryl, Cyano-pyridyl, Pyrryl-phenyl, Niederalkoxycarbonylamino-niederalkyl-pyridyl, N'-Cycloalkyl-ureido-phenyl, (Benzimidazol-2-on)-yl, Phenylniederalkoxyphenyl oder Hydroxyphenyl und $R_1$ für Niederalkyl, Phenylniederalkyl, Phenoxyniederalkyl oder Pyridyloxyniederalkyl steht und $R_2$ Niederalkyl, Phenylniederalkyl, Phenoxyniederalkyl oder Phenyl bedeutet, oder solche Verbindungen als freie Basen.

13. Optisch aktive Verbindungen der Formel III gemäß Anspruch 11, worin X Chlor oder Brom oder den Rest der Schwefelsäure oder Phosphorsäure bedeutet, $Ar_1$ für 1-Naphthyl, 3-Niederalkyl-phenyl, 2-Niederalkenyl-phenyl, 2-Niederalkenyloxy-phenyl, 4-Niederalkanoylamino-phenyl, 4-(2-Niederalkoxy-äthyl)-phenyl, 2-Pyrazinyl, 2-Pyrimidinyl, (1-Pyrryl)-phenyl, 4-Indolyl, 5-(2-Niederalkoxycarbonylamino-äthyl)-pyridyl, 3-Cyano-pyridyl, 4-(Benzimidazol-2-on)-yl, 4-(N'-Cycloalkyl-ureido)-phenyl, Benzyloxy-phenyl, Hydroxyphenyl und $R_1$ für Niederalkyl, Phenylniederalkyl oder Phenoxyniederalkyl steht, und $R_2$ Niederalkyl, Phenylniederalkyl oder Phenyl bedeutet, oder solche Verbindungen als freie Basen.

14. Optisch aktive Verbindungen der Formel III gemäß Anspruch 11, worin $X^{\ominus}$ Chlor oder das Anion der Schwefelsäure bedeutet, $Ar_1$ für 4-Benzyloxyphenyl, 4-Hydroxyphenyl, 2-Allyloxyphenyl oder 1-Naphthyl und $R_1$ für Niederalkyl steht und $R_2$ Methyl darstellt, oder solche Verbindungen als freie Basen.

**Patentansprüche für den Vertragsstaat AT**

**gültig für das als Vertragsstaat benannte Österreich**

1. Verfahren zur Umkehrung der Konfiguration in optisch aktiven Verbindungen der Formel

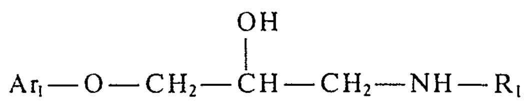

(I)

worin $Ar_1$ für einen gegebenenfalls substituierten mono- oder polycyclischen, carbo- oder heterocyclischen Rest steht, der mindestens einen Ring mit aromatischem Charakter aufweist und der über ein Ringkohlenstoffatom, vorzugsweise des Rings mit aromatischem Charakter, mit dem Sauerstoffatom verbunden ist, $R_1$ einen gegebenenfalls substituierten aliphatischen, cycloaliphatischen oder araliphatischen Kohlenwasserstoffrest bedeutet, oder deren Salzen, dadurch gekennzeichnet, daß man eine optisch aktive Verbindung der Formel

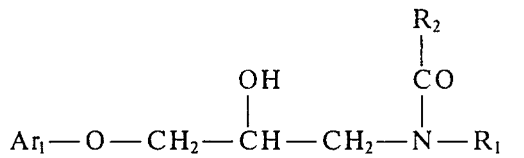

(II)

mit der R(+)- bzw. der S(−)-Konfiguration, worin $R_2$ einen gegebenenfalls substituierten mono- oder polycyclischen, carbo- oder heterocyclischen Rest oder einen gegebenenfalls substituierten aliphatischen, cycloaliphatischen oder araliphatischen Kohlenwasserstoffrest darstellt, durch Behandeln mit einer starken, sauerstoffhaltigen anorganischen oder organischen Säure oder deren Halogeniden in eine optisch aktive Verbindung der Formel

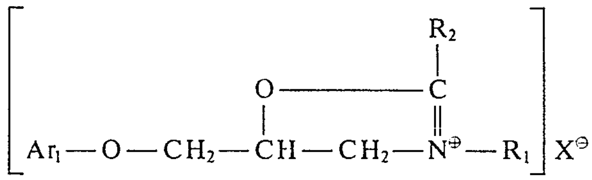

(III)

mit der R(−)- bzw. der S(+)-Konfiguration, worin $X^{\ominus}$ für das Anion einer starken, sauerstoffhaltigen anorganischen oder organischen Säure oder eines Halogenatoms steht, umwandelt, und die so erhaltene Verbindung der Formel III, gegebenenfalls über die entsprechende freie Base als Zwischenstufe, zu einer Verbindung der Formel I mit einer Konfiguration, die derjenigen des verwendeten Ausgangsmaterials entgegengesetzt ist, hydrolysiert, und wenn erwünscht, eine freie Verbindung der Formel I in ein Salz oder ein erhaltenes Salz in die freie Verbindung überführt.

2. Verfahren nach Anspruch 1, dadurch gekennzeichnet, daß man als starke, sauerstoffhaltige anorganische oder organische Säure konzentrierte Schwefelsäure, konzentrierte Phosphorsäure, Phosphorpentoxid oder eine starke organische Sulfonsäure verwendet.

3. Verfahren nach Anspruch 1, dadurch gekennzeichnet, daß man als Halogenid einer starken, sauerstoffhaltigen anorganischen oder organischen Säure Thionylchlorid, Thionylbromid, Sulfurylchlorid, Chlorsulfonsäure, Methansulfonylchlorid, Phosphortrichlorid, Phosphoroxychlorid oder Phosphorpentachlorid verwendet.

4. Verfahren nach Anspruch 1−2, dadurch gekennzeichnet, daß man bei Verwendung einer starken, sauerstoffhaltigen anorganischen oder organischen Säure in einem Temperaturbereich von +50 bis +200° arbeitet.

5. Verfahren nach Anspruch 1 oder 3, dadurch gekennzeichnet, daß man bei Verwendung eines Halogenids einer starken, sauerstoffhaltigen anorganischen oder organischen Säure in einem Temperaturbereich von −10 bis +70° arbeitet.

6. Verfahren nach Anspruch 1, dadurch gekennzeichnet, daß man die Hydrolyse in einem sauren oder basischen Medium durchführt.

7. Verfahren nach einem der Ansprüche 1−6, dadurch gekennzeichnet, daß man einen optisch aktiven Ausgangsstoff der Formel II, worin $Ar_1$ für Naphthyl, Niederalkyl-phenyl, Niederalkenyl-phenyl, Niederalkenyloxy-phenyl, Niederalkanoylamino-phenyl, Niederalkoxyniederalkyl-phenyl, 9,10-Äthano-9,10-dihydro-benzanthryl, Pyrazinyl, Pyrimidinyl, Indolyl, Benzofuryl, Cyano-pyridyl, Pyrrylphenyl, Niederalkoxycarbonylaminoniederalkyl-pyridyl, N'-Cycloalkylureido-phenyl, (Benzimidazol-2-on)-yl, Phenylniederalkoxy-phenyl oder Hydroxyphenyl und $R_1$ für Niederalkyl, Phenylniederalkyl, Phenoxyniederalkyl oder Pyridyloxyniederalkyl steht und $R_2$ Niederalkyl, Phenylniederalkyl, Phenoxyniederalkyl oder Phenyl bedeutet, mit konzentrierter Phosphorsäure oder konzentrierter

Schwefelsäure oder einem Halogenid solcher Säuren umsetzt und die so erhaltene Verbindung der Formel III, gegebenenfalls über die entsprechende freie Base als Zwischenstufe, zur entsprechenden Verbindung der Formel I mit entgegengesetzter Konfiguration hydrolysiert.

8. Verfahren nach einem der Ansprüche 1—6, dadurch gekennzeichnet, daß man einen optisch aktiven Ausgangsstoff der Formel II, worin $Ar_1$ für 1-Naphthyl, 3-Niederalkyl-phenyl, 2-Niederalkenyl-phenyl, 2-Niederalkenyloxy-phenyl, 4-Niederalkanoylamino-phenyl, 4-(2-Niederalkoxyäthyl)-phenyl, 2-Pyrazinyl, 2-Pyrimidinyl, (1-Pyrryl)-phenyl, 4-Indolyl, 5-(2-Niederalkoxycarbonylamino-äthyl)-pyridyl, 3-Cyano-pyridyl, 4-(Benzimidazol-2-on)-yl, 4-(N'-Cycloalkyl-ureido)-phenyl, Benzyloxy-phenyl oder Hydroxy-phenyl und $R_1$ für Niederalkyl, Phenylniederalkyl oder Phenoxyniederalkyl steht, und $R_2$ Niederalkyl, Phenylniederalkyl oder Phenyl bedeutet, mit konzentrierter Phosphorsäure oder konzentrierter Schwefelsäure oder einem Chlorid oder Bromid solcher Säuren umsetzt und die so erhaltene Verbindung der Formel III, gegebenenfalls über die entsprechende freie Base als Zwischenstufe, zur entsprechenden Verbindung der Formel I mit entgegengesetzter Konfiguration hydrolysiert.

9. Verfahren nach einem der Ansprüche 1—6, dadurch gekennzeichnet, daß man einen optisch aktiven Ausgangsstoff der Formel II, in dem $Ar_1$ für 1-Naphthyl, 3-Methyl-phenyl, 2-Allyl-phenyl, 2-Allyloxy-phenyl, 4-Acetylamino-phenyl, 4-Indolyl, 4-(2-methoxyäthyl)-phenyl, 2-Pyrazinyl, 2-Pyrimidinyl, 2-(1-Pyrryl)-phenyl, 4-(N'-Cyclohexyl-ureido)-phenyl, 4-Benzyloxy-phenyl oder 4-Hydroxy-phenyl, $R_1$ für Niederalkyl oder Phenylniederalkyl und $R_2$ für Niederalkyl, Phenylniederalkyl oder Phenyl steht, mit konzentrierter Phosphorsäure oder konzentrierter Schwefelsäure oder einem Chlorid oder Bromid solcher Säuren umsetzt und die so erhaltene Verbindung der Formel III, gegebenenfalls über die entsprechende freie Base als Zwischenstufe, mittels wässeriger Mineralsäure oder wässeriger Alkalilauge zur entsprechenden Verbindung der Formel I mit entgegengesetzter Konfiguration hydrolysiert.

10. Verfahren nach einem der Ansprüche 1—6, dadurch gekennzeichnet, daß man einen optisch aktiven Ausgangsstoff der Formel II, worin $Ar_1$ für 2-(1-Pyrryl)-phenyl, 1-Naphthyl, 2-Allyl-phenyl, 2-Allyloxy-phenyl, 4-(N'-Cyclo-hexylureido)-phenyl, 2-Pyrazinyl, 4-Benzyloxy-phenyl oder 4-Hydroxy-phenyl, $R_1$ für Niederalkyl und $R_2$ für Niederalkyl oder Phenyl steht, mit konzentrierter Schwefelsäure oder einem Chlorid davon oder mit Thionylchlorid umsetzt und die so erhaltene Verbindung der Formel III, gegebenenfalls über die entsprechende freie Base als Zwischenstufe, mittels wässeriger Salzsäure oder Schwefelsäure oder mittels wässeriger Alkalilauge zur entsprechenden Verbindung der Formel I mit entgegengesetzter Konfiguration hydrolysiert.

11. Verfahren zur Herstellung optisch aktiver Verbindungen der Formel

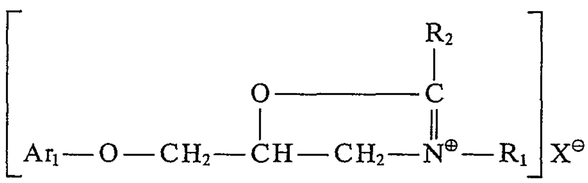

(III)

mit der R(—)- bzw. der S(+)-Konfiguration, worin $X^{\ominus}$ für das Anion einer starken, sauerstoffhaltigen anorganischen oder organischen Säure oder eines Halogenatoms steht und $Ar_1$, $R_1$ und $R_2$ die im Anspruch 1 angegebene Bedeutung haben, dadurch gekennzeichnet, daß man eine optisch aktive Verbindung der Formel

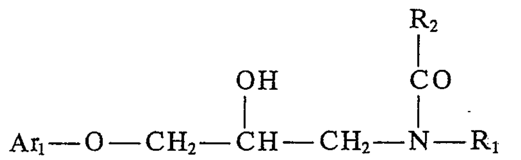

(II)

mit der R(+)- bzw. der S(—)-Konfiguration, worin $Ar_1$, $R_1$ und $R_2$ die im Anspruch 1 angegebene Bedeutung haben, mit einer starken, sauerstoffhaltigen anorganischen oder organischen Säure oder deren Halogeniden behandelt.

**Claims for the contracting States: BE, CH, DE, FR, GB, IT, LU, NL, SE**

1. Process for inverting the configuration in optically active compounds of the formula

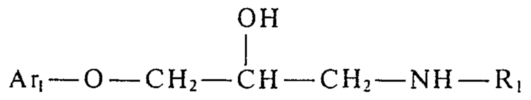

(I)

in which

$Ar_1$ represents an optionally substituted monocyclic or polycyclic, carbocyclic or heterocyclic radial that has at least one ring of aromatic character and is bonded to the oxygen atom by way of a ring carbon atom, preferably of the ring of aromatic character, and

$R_1$ represents an optionally substituted aliphatic, cycloaliphatic or araliphatic hydrocarbon radical

or the salts thereof, characterised in that an optically active compound of the formula

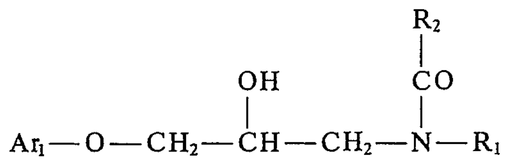

(II)

having the R(+) or S(−) configuration, in which

$R_2$ represents an optionally substituted monocyclic or polycyclic, carbocyclic or heterocyclic radical or an optionally substituted aliphatic, cycloaliphatic or araliphatic hydrocarbon radical,

is converted, by treating with a strong oxygen-containing inorganic or organic acid or halides thereof, into an optically active compound of the formula

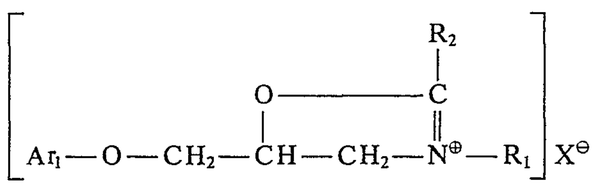

(III)

having the R(−) or S(+) configuration, in which

$X^{\ominus}$ represents the anion of a strong, oxygen-containing inorganic or organic acid or of a halogen atom

and the resulting compound of the formula III is hydrolysed, optionally by way of the corresponding free base as intermediate, to form a compound of the formula I of a configuration opposite to that of the starting material used and, if desired, a free compound of the formula I is converted into a salt or a resulting salt is converted into the free compound.

2. Process according to claim 1, characterised in that there is used as strong, oxygen-containing inorganic or organic acid concentrated sulphuric acid, concentrated phosphoric acid, phosphorus pentoxide or a strong organic sulphonic acid.

3. Process according to claim 1, characterised in that there is used as a halide of a strong, oxygen-containing inorganic or organic acid thionyl chloride, thionyl bromide, sulphuryl chloride, chlorosulphonic acid, methanesulphonyl chloride, phosphorus trichloride, phosphorus oxychloride or phosphorus pentachloride.

4. Process according to claim 1−2, characterised in that when using a strong, oxygen-containing inorganic or organic acid, the process is carried out in a temperature range of +50 to +200° C.

5. Process according to claim 1 or 3, characterised in that when using a halide of a strong, oxygen-containing inorganic or organic acid, the process is carried out in a temperature range of −10 to +70° C.

6. Process according to claim 1, characterised in that the hydrolysis is carried out in an acidic or basic medium.

7. Process according to one of the claims 1−6, characterised in that an optically active starting material of the formula II, in which

$Ar_1$ represents naphthyl, lower alkylphenyl, lower alkenylphenyl, lower alkenyloxyphenyl, lower alkanoylaminophenyl, lower alkoxy-lower alkylphenyl, 9,10-ethano-9,10-dihydrobenz-anthryl,

pyrazinyl, pyrimidinyl, indolyl, benzofuryl, cyanopyridyl, pyrrylphenyl, lower alkoxycarbonylamino-lower alkylpyridyl, N'-cycloalkylureidophenyl, (benzimidazol-2-on)yl, phenyl-lower alkoxyphenyl or hydroxyphenyl,

$R_1$ represents lower alkyl, phenyl-lower alkyl, phenoxy-lower alkyl or pyridyloxy-lower alkyl, and

$R_2$ represents lower alkyl, phenyl-lower alkyl, phenoxy-lower alkyl or phenyl,

is reacted with concentrated phosphoric acid or concentrated sulphuric acid or a halide of such acids, and the resulting compound of the formula III is hydrolysed, optionally by way of the corresponding free base as intermediate, to form the corresponding compound of the formula I with an opposite configuration.

8. Process according to one of the claims 1—6, characterised in that an optically active starting material of the formula II, in which

$Ar_1$ represents 1-naphthyl, 3-lower alkylphenyl, 2-lower alkenylphenyl, 2-lower alkenyloxyphenyl, 4-lower alkanoylaminophenyl, 4-(2-lower alkoxyethyl)phenyl, pyrazin-2-yl, pyrimidin-2-yl, (1-pyrryl)-phenyl, 4-indolyl, 5-(2-lower alkoxycarbonylaminoethyl)pyridyl, 3-cyanopyridyl, 4-(benzimidazol-2-on)yl, 4-(N'-cycloalkylureido)phenyl, benzyloxyphenyl or hydroxyphenyl,

$R_1$ represents lower alkyl, phenyl-lower alkyl or phenoxy-lower alkyl, and

$R_2$ represents lower alkyl, phenyl-lower alkyl or phenyl,

is reacted with concentrated phosphoric acid or concentrated sulphuric acid or a chloride or bromide of such acids, and the resulting compound of the formula III is hydrolysed, optionally by way of the corresponding free base as intermediate, to form the corresponding compound of the formula I with an opposite configuration.

9. Process according to one of the claims 1—6, characterised in that an optically active starting material of the formula II, in which

$Ar_1$ represents 1-naphthyl, 3-methylphenyl, 2-allylphenyl, 2-allyloxyphenyl, 4-acetylaminophenyl, 4-indolyl, 4-(2-methoxyethyl)phenyl, pyrazin-2-yl, pyrimidin-2-yl, 2-(1-pyrryl)phenyl, 4-(N'-cyclohexylureido)phenyl, 4-benzyloxyphenyl or 4-hydroxyphenyl,

$R_1$ represents lower alkyl or phenyl-lower alkyl and

$R_2$ represents lower alkyl, phenyl-lower alkyl or phenyl,

is reacted with concentrated phosphoric acid or concentrated sulphuric acid or a chloride or bromide of such acids, and the resulting compound of the formula III is hydrolysed, optionally by way of the corresponding free base as intermediate, by means of aqueous mineral acid or aqueous alkali metal hydroxide solution to form the corresponding compound of the formula I with an opposite configuration.

10. Process according to one of the claims 1—6, characterised in that an optically active starting material of the formula II, in which

$Ar_1$ represents 2-(1-pyrryl)phenyl, 1-naphthyl, 2-allylphenyl, 2-allyloxyphenyl, 4-(N'-cyclohexylureido)phenyl, pyrazin-2-yl, 4-benzyloxyphenyl or 4-hydroxyphenyl,

$R_1$ represents lower alkyl and

$R_2$ represents lower alkyl or phenyl,

is reacted with concentrated sulphuric acid or a chloride thereof, or with thionyl chloride, and the resulting compound of the formula III is hydrolysed, optionally by way of the corresponding free base as intermediate, by means of aqueous hydrochloric acid or sulphuric acid or by means of aqueous alkali metal hydroxide solution, to form the corresponding compound of the formula I with an opposite condifuration.

11. Optically active compounds of the formula

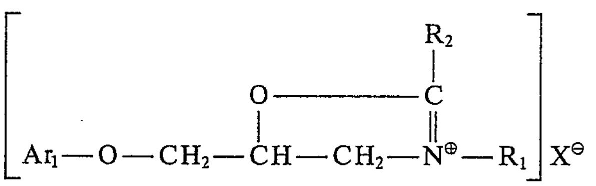

(III)

having the R(—) or S(+) configuration, in which

$X^\ominus$ represents the anion of a strong, oxygen-containing inorganic or organic acid or of a halogen atom and

$Ar_1$, $R_1$ and $R_2$ have the meanings given above, or such compounds in the form of free bases.

12. Optically active compounds of the formula III according to claim 11, in which

X represents a halogen atom or the radical of sulphuric or phosphoric acid,
$Ar_1$ represents naphthyl, lower alkylphenyl, lower alkenylphenyl, lower alkenyloxyphenyl, lower alkanoylaminophenyl, lower alkoxy-lower alkylphenyl, 9,10-ethano-9,10-dihydrobenz-anthryl, pyrazinyl, pyrimidinyl, indolyl, benzofuryl, cyanopyridyl, pyrrylphenyl, lower alkoxycarbonylamino-lower alkylpyridyl, N'-cycloalkylureidophenyl, (benzimidazol-2-on)yl, phenyl-lower alkoxyphenyl or hydroxyphenyl,
$R_1$ represents lower alkyl, phenyl-lower alkyl, phenoxy-lower alkyl or pyridyloxy-lower alkyl, and
$R_2$ represents lower alkyl, phenyl-lower alkyl, phenoxy-lower alkyl or phenyl,

or such compounds in the form of free bases.

13. Optically active compounds of the formula III according to claim 11, in which

X represents chlorine or bromine or the radical of sulphuric or phosphoric acid,
$Ar_1$ represents 1-naphthyl, 3-lower alkylphenyl, 2-lower alkenylphenyl, 2-lower alkenyloxyphenyl, 4-lower alkanoylaminophenyl, 4-(2-lower alkoxyethyl)phenyl, pyrazin-2-yl, pyrimidin-2-yl, (1-pyrryl)phenyl, 4-indolyl, 5-(2-lower alkoxycarbonylaminoethyl)pyridyl, 3-cyanopyridyl, 4-(benzimidazol-2-on)yl, 4-(N'-cycloalkylureido)phenyl, benzyloxyphenyl or hydroxyphenyl,
$R_1$ represents lower alkyl, phenyl-lower alkyl or phenoxy-lower alkyl, and
$R_2$ represents lower alkyl, phenyl-lower alkyl or phenyl,

or such compounds in the form of free bases.

14. Optically active compounds of the formula III according to claim 11, in which

$X^{\ominus}$ represents chlorine or the anion of sulfuric acid,
$Ar_1$ represents 4-benzyloxyphenyl, 4-hydroxyphenyl, 2-allyloxyphenyl or 1-naphthyl,
$R_1$ represents lower alkyl, and
$R_2$ represents methyl,

or such compounds in the form of free bases.

**Claims for the contracting State: AT**

1. Process for inverting the configuration in optically active compounds of the formula

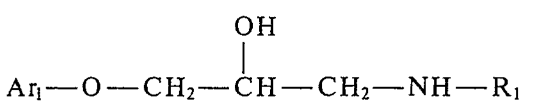
(I)

in which

$Ar_1$ represents an optionally substituted monocyclic or polycyclic, carbocyclic or heterocyclic radical that has at least one ring of aromatic character and is bonded to the oxygen atom by way of a ring carbon atom, preferably of the ring of aromatic character, and
$R_1$ represents an optionally substituted aliphatic, cycloaliphatic or araliphatic hydrocarbon radical,

or the salts thereof, characterised in that an optically active compound of the formula

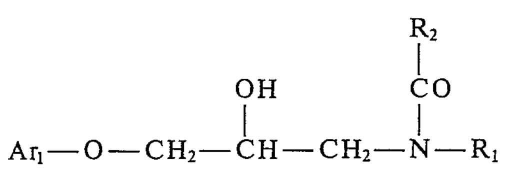
(II)

having the R(+) or S(−) configuration, in which

$R_2$ represents an optionally substituted monocyclic or polycyclic, carbocyclic or heterocyclic radical or an optionally substituted aliphatic, cycloaliphatic or araliphatic hydrocarbon radical,

is converted, by treating with a strong oxygen-containing inorganic or organic acid or halides thereof, into an optically actice compound of the formula

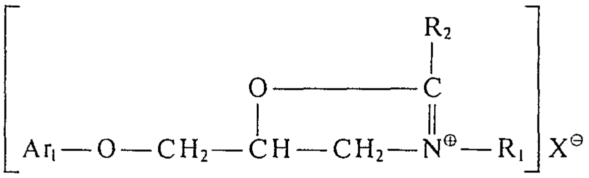

(III)

having the R(−) or S(+) configuration, in which

$X^{\ominus}$ represents the anion of a strong, oxygen-containing inorganic or organic acid or of a halogen atom

and the resulting compound of the formula III is hydrolysed, optionally by way of the corresponding free base as intermediate, to form a compound of the formula I of a configuration opposite to that of the starting material used and, if desired, a free compound of the formula I is converted into a salt or a resulting salt is converted into the free compound.

2. Process according to claim 1, characterised in that there is used as strong, oxygen-containing inorganic or organic acid concentrated sulphuric acid, concentrated phosphoric acid, phosphorus pentoxide or a strong organic sulphonic acid.

3. Process according to claim 1, characterised in that there is used as a halide of a strong, oxygen-containing inorganic or organic acid thionyl chloride, thionyl bromide, sulphuryl chloride, chlorosulphonic acid, methanesulphonyl chloride, phosphorus trichloride, phosphorus oxychloride or phosphorus pentachloride.

4. Process according to claim 1−2, characterised in that when using a strong, oxygen-containing inorganic or organic acid, the process is carried out in a temperature range of +50 to +200° C.

5. Process according to claim 1 or 3, characterised in that when using a halide of a strong, oxygen-containing inorganic or organic acid, the process is carried out in a temperature range of −10 to +70° C.

6. Process according to claim 1, characterised in that the hydrolysis is carried out in an acidic or basic medium.

7. Process according to one of the claims 1−6, characterised in that an optically active starting material of the formula II, in which

$Ar_1$ represents naphthyl, lower-alkylphenyl, lower alkenylphenyl, lower alkenyloxyphenyl, lower alkanoylaminophenyl, lower alkoxy-lower alkylphenyl, 9,10-ethano-9,10-dihydrobenz-anthryl, pyrazinyl, pyrimidinyl, indolyl, benzofuryl, cyanopyridyl, pyrrylphenyl, lower alkoxycarbonylamino-lower alkylpyridyl, N′-cycloalkylureidophenyl, (benzimidazol-2-on)yl, phenyl-lower alkoxyphenyl or hydroxyphenyl,
$R_1$ represents lower alkyl, phenyl-lower alkyl, phenoxy-lower alkyl or pyridyloxy-lower alkyl, and
$R_2$ represents lower alkyl, phenyl-lower alkyl, phenoxy-lower alkyl or phenyl,

is reacted with concentrated phosphoric acid or concentrated sulphuric acid or a halide of such acids, and the resulting compound of the formula III is hydrolysed, optionally by way of the corresponding free base as intermediate, to form the corresponding compound of the formula I with an opposite configuration.

8. Process according to one of the claims 1−6, characterised in that an optically active starting material of the formula II, in which

$Ar_1$ represents 1-naphthyl, 3-lower alkylphenyl, 2-lower alkenylphenyl, 2-lower alkenyloxyphenyl, 4-lower alkanoylaminophenyl, 4-(2-lower alkoxyethyl)phenyl, pyrazin-2-yl, pyrimidin-2-yl, (1-pyrryl)phenyl, 4-indolyl, 5-(2-lower alkoxycarbonylaminoethyl)pyridyl, 3-cyanopyridyl, 4-(benzimidazol-2-on)yl, 4-(N′-cycloalkylureido)phenyl, benzyloxyphenyl or hydroxyphenyl,
$R_1$ represents lower alkyl, phenyl-lower alkyl or phenoxy-lower alkyl, and
$R_2$ represents lower alkyl, phenyl-lower alkyl or phenyl,

is reacted with concentrated phosphoric acid or concentrated sulphuric acid or a chloride or bromide of such acids, and the resulting compound of the formula III is hydrolysed, optionally by way of the corresponding free base as intermediate, to form the corresponding compound of the formula I with an opposite configuration.

9. Process according to one of the claims 1−6, characterised in that an optically active starting material of the formula II, in which

$Ar_1$ represents 1-naphthyl, 3-methylphenyl, 2-allylphenyl, 2-allyloxyphenyl, 4-acetylaminophenyl, 4-indolyl, 4-(2-methoxyethyl)phenyl, pyrazin-2-yl, pyrimidin-2-yl, 2-(1-pyrryl)phenyl, 4-(N′-cyclohexylureido)phenyl, 4-benzyloxyphenyl or 4-hydroxyphenyl,

$R_1$ represents lower alkyl or phenyl-lower alkyl and
$R_2$ represents lower alkyl, phenyl-lower alkyl or phenyl,

is reacted with concentrated phosphoric acid or concentrated sulphuric acid or a chloride or bromide of such acids, and the resulting compound of the formula III is hydrolysed, optionally by way of the corresponding free base as intermediate, by means of aqueous mineral acid or aqueous alkali metal hydroxide solution to form the corresponding compound of the formula I with an opposite configuration.

10. Process according to one of the claims 1–6, characterised in that an optically active starting material of the formula II, in which

$Ar_1$ represents 2-(1-pyrryl)phenyl, 1-naphthyl, 2-allylphenyl, 2-allyloxyphenyl, 4-(N'-cyclohexylureido)phenyl, pyrazin-2-yl, 4-benzyloxyphenyl or 4-hydroxyphenyl,
$R_1$ represents lower alkyl, and
$R_2$ represents lower alkyl or phenyl,

is reacted with concentrated sulfuric acid or a chloride thereof, or with thionyl chloride, and the resulting compound of the formula III is hydrolysed, optionally by way of the corresponding free base as intermediate, by means of aqueous hydrochloric acid or sulfuric acid or by means of aqueous alkali metal hydroxide solution, to form the corresponding compound of the formula I with an opposite configuration.

11. Process for producing optically active compounds of the formula

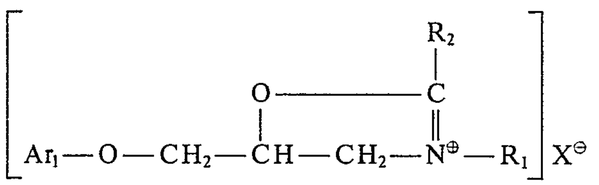 (III)

having the R(–) or S(+) configuration, in which

$X^{\ominus}$ represents the anion of a strong, oxygen-containing inorganic or organic acid or of a halogen atom,

and $Ar_1$, $R_1$ and $R_2$ have the meanings defined in claim 1, characterised in that an optically active compound of the formula

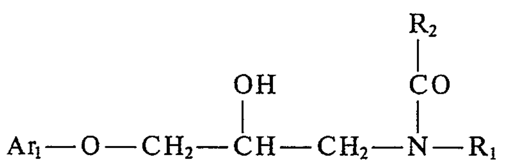 (II)

having the R(+) or S(–) configuration, in which $Ar_1$, $R_1$ and $R_2$ have the meanings defined in claim 1, is treated with a strong, oxygen-containing, inorganic or organic acid or with halides thereof.

**Revendications pour les Etats contractants: BE, CH, DE, FR, GB, IT, LU, NL, SE**

1. Procédé pour inverser la configuration de composés énantiomères de formule

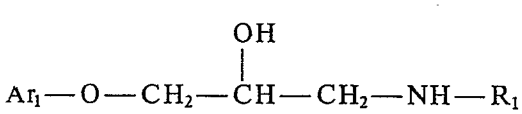 (I)

dans laquelle $Ar_1$ représente un reste mono- ou poly-cyclique carbocyclique ou hétérocyclique éventuellement substitué contenant au moins un cycle à caractère aromatique, et qui est relié à l'atome d'oxygène par l'intermédiaire d'un atome de carbone cyclique, de préférence du cycle à caractère aromatique, $R_1$ représente un reste hydrocarboné aliphatique, cycloaliphatique ou araliphatique éventuellement substitué, ou de leurs sels, caractérisé en ce que l'on convertit un énantiomère de formule

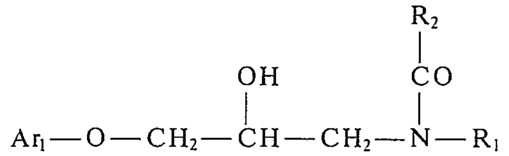

(II)

en configuration R(+) ou S(−) respectivement, $R_2$ représentant un reste mono- ou poly-cyclique carbocyclique ou hétérocyclique éventuellement substitué, ou un reste hydrocarboné aliphatique, cycloaliphatique ou araliphatique éventuellement substitué par traitement à l'aide d'un acide organique ou minéral oxygéné fort ou d'un halogénure d'un tel acide, en un énantiomère de formule

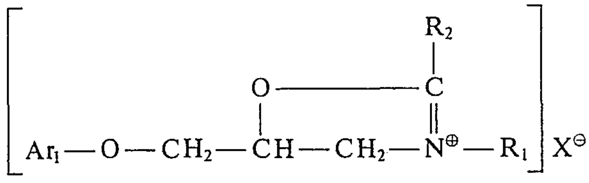

(III)

en configuration R(−) ou S(+) respectivement, $X^{\ominus}$ représentant l'anion d'un acide organique ou minéral oxygéné fort ou un atome d'halogène, et on hydrolyse le composé de formule III ainsi obtenu, en passant le cas échéant par l'intermédiaire de la base libre correspondante, en un composé de formule I dont la configuration est opposée à celle du produit de départ mis en oeuvre, après quoi, si on le désire, on convertit un composé de formule I en un sel ou un sel obtenu en le composé libre.

2. Procédé selon la revendication 1, caractérisé en ce que l'on utilise en tant qu'acides organiques ou minéraux oxygénés forts de l'acide sulfurique concentré, de l'acide phosphorique concentré, de l'anhydride phosphorique ou un acide organique sulfonique fort.

3. Procédé selon la revendication 1, caractérisé en ce que l'on utilise en tant qu'halogénure d'un acide organique ou minéral oxygéné fort le chlorure de thionyle, le bromure de thionyle, le chlorure de sulfuryle, l'acide chlorosulfonique, le chlorure de méthane-sulfonyle, le trichlorure de phosphore, l'oxychlorure de phosphore ou le pentachlorure de phosphore.

4. Procédé selon les revendications 1 et 2, caractérisé en ce que, lorsqu'on utilise un acide organique ou minéral oxygéné fort, on opère dans l'intervalle de température de +50 à +200° C.

5. Procédé selon la revendication 1 ou 3, caractérisé en ce que, lorsqu'on utilise un halogénure d'un acide organique ou minéral oxygéné fort, on opère dans un intervalle de température de −10 à 70° C.

6. Procédé selon la revendication 1, caractérisé en ce que l'on effectue l'hydrolyse en milieu acide ou basique.

7. Procédé selon l'une des revendications 1 à 6, caractérisé en ce que l'on fait réagir un énantiomère de formule II dans laquelle $Ar_1$ représente un groupe naphtyle, alkyle inférieur-phényle, alcényle inférieur-phényle, alcényloxy inférieur-phényle, alcanoylamino inférieur-phényle, alcoxy inférieur-alkyle inférieur-phényle, 9,10-éthano-9,10-dihydro-benzanthryle, pyrazinyle, pyrimidinyle, indolyle, benzofuryle, cyanopyridyle, pyrryl-phényle, alcoxy inférieur-carbonylamino-alkyle inférieur-pyridyle, N'-cycloalkyluréido-phényle, (benzimidazole-2-one)-yle, phényl-alcoxy inférieur-phényle ou hydroxy-phényle et $R_1$ représente un groupe alkyle inférieur, phényl-alkyle inférieur, phénoxy-alkyle inférieur ou pyridyloxy-alkyle inférieur, et $R_2$ représente un groupe alkyle inférieur, phényl-alkyle inférieur, phénoxy-alkyle inférieur ou phényle, avec l'acide phosphorique concentré ou l'acide sulfurique concentré ou un halogénure de ces acides, et on hydrolyse le composé de formule III ainsi obtenu, le cas échéant en passant par l'intermédiaire de la base libre correspondante, en le composé correspondant de formule I à configuration opposée.

8. Procédé selon l'une des revendications 1 à 6, caractérisé en ce que l'on fait réagir un énantiomère de départ de formule II dans laquelle $Ar_1$ représente un groupe 1-naphtyle, 3-alkyle inférieur-phényle, 2-alcényle inférieur-phényle, 2-alcényloxy inférieur-phényle, 4-alcanoylamino inférieur-phényle, 4-(2-alcoxy inférieur-éthyl)-phényle, 2-pyrazinyle, 2-pyrimidinyle, (1-pyrryl)-phényle, 4-indolyle, 5-(2-alcoxy inférieur-carbonylamino-éthyl)-pyridyle, 3-cyanopyridyle, 4-(benzimidazole-2-one)-yle, 4-(N'-cycloalkyluréido)-phényle, benzyloxyphényle ou hydroxyphényle et $R_1$ représente un groupe alkyle inférieur, phénylalkyle inférieur ou phénoxyalkyle inférieur, et $R_2$ représente un groupe alkyle inférieur, phénylalkyle inférieur ou phényle, avec l'acide phosphorique concentré ou l'acide sulfurique concentré ou un chlorure ou bromure de ces acides, et on hydrolyse le composé de formule III ainsi obtenu, le cas échéant en passant par l'intermédiaire de la base libre correspondante, en le composé correspondant de formule I à configuration opposée.

9. Procédé selon l'une des revendications 1 à 6, caractérisé en ce que l'on fait réagir un énantiomère de formule II dans laquelle $Ar_1$ représente un groupe 1-naphtyle, 3-méthylphényle, 2-allylphényle, 2-allyloxyphenyle, 4-acétylaminophényle, 4-indolyle, 4-(2-méthoxyéthyl)-phenyle, 2-pyrazinyle, 2-pyrimidinyle, 2-(1-pyrryl)-phényle, 4-(N'-cyclohexyluréido)-phényle, 4-benzyloxyphényle ou 4-hydroxyphényle, $R_1$ représente un groupe alkyle inférieur ou phénylalkyle inférieur et $R_2$ représente un groupe alkyle inférieur, phénylalkyle inférieur ou phényle, avec l'acide phosphorique concentré ou l'acide

sulfurique concentré ou un chlorure ou bromure de ces acides et on hydrolyse le composé de formule III ainsi obtenu, le cas échéant en passant par l'intermédiaire de la base libre correspondante, à l'aide d'un acide minéral aqueux ou d'une lessive alcaline aqueuse, en le composé correspondant de formule I à configuration opposée.

10. Procédé selon l'une des revendications 1 à 6, caractérisé en ce que l'on fait réagir un énantiomère de formule II dans laquelle $Ar_1$ représente un groupe 2-(1-pyrryl)-phényle, 1-naphtyle, 2-allylphényle, 2-allyloxyphényle, 4-(N'-cyclohexyluréido)-phényle, 2-pyrazinyle, 4-benzyloxyphényle ou 4-hydroxyphényle, $R_1$ représente un groupe alkyle inférieur et $R_2$ un groupe alkyle inférieur ou phényle, avec l'acide sulfurique concentré ou un chlorure de cet acide, ou avec le chlorure de thionyle, et on hydrolyse le composé de formule III ainsi obtenu, le cas échéant en passant par l'intermédiaire de la base libre correspondante, à l'aide d'acide chlorhydrique ou sulfurique aqueux ou à l'aide d'une lessive alcaline aqueuse, en le composé correspondant de formule I à configuration opposée.

11. Enantiomère de formule

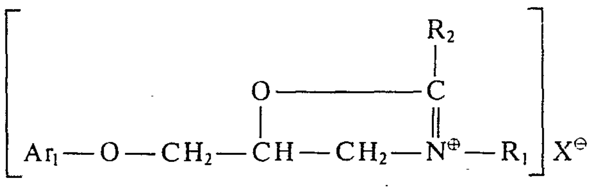

(III)

en configuration R(−) ou S(+) respectivement, dans laquelle $X^{\ominus}$ représente l'anion d'un acide organique ou minéral oxygéné fort ou un atome d'halogène et $Ar_1$, $R_1$ et $R_2$ ont les significations indiquées dans la revendication 1, ou les mêmes composés à l'état de base libre.

12. Enantiomères de formule III selon la revendication 11, dans lesquels X représente un halogène ou le reste de l'acide sulfurique ou de l'acide phosphorique, $Ar_1$ représente un groupe naphtyle, alkyle inférieur-phényle, alcényle inférieur-phényle, alcényloxy inférieur-phényle, alcanoylamino inférieur-phényle, alcoxy inférieur-alkyle inférieur-phényle, 9,10-éthano-9,10-dihydro-benzanthryle, pyrazinyle, pyrimidinyle, indolyle, benzofuryle, cyanopyridyle, pyrryl-phényle, alcoxy inférieur-carbonylamino-alkyle inférieur-pyridyle, N'-cycloalkyluréido-phényle, (benzimidazole-2-one)-yle, phényl-alcoxy inférieur-phényle, hydroxyphényle, $R_1$ représente un groupe alkyle inférieur, phénylalkyle inférieur, phénoxyalkyle inférieur ou pyridyloxy-alkyle inférieur et $R_2$ représente un groupe alkyle inférieur, phénylalkyle inférieur, phénoxyalkyle inférieur ou phényle, ou les mêmes composés à l'état de base libre.

13. Enantiomères de formule III selon la revendication 11, dans lesquels X représente le chlore ou le brome ou le reste de l'acide sulfurique ou de l'acide phosphorique, $Ar_1$ représente un groupe 1-naphtyle, 3-alkyle inférieur-phényle, 2-alcényle inférieur-phényle, 2-alcényloxy inférieur-phényle, 4-alcanoylamino inférieur-phényle, 4-(2-alcoxy inférieur-éthyl)-phényle, 2-pyrazinyle, 2-pyrimidinyle, (1-pyrryl)-phényle, 4-indolyle, 5-(2-alcoxy inférieur-carbonylamino-éhtyl)-pyridyle, 3-cyano-pyridyle, 4-(benzimidazole-2-one)-yle, 4-(N'-cycloalkyluréido)-phényle, benzyloxyphényle, hydroxyphényle et $R_1$ représente un groupe alkyle inférieur, phénylalkyle inférieur ou phénoxyalkyle inférieur, et $R_2$ représente un groupe alkyle inférieur, phénylalkyle inférieur ou phényle, ou les mêmes composés à l'état de base libre.

14. Enantiomères de formule III selon la revendication 11, dans lesquels $X^{\ominus}$ représente le chlore ou l'anion de l'acide sulfurique, $Ar_1$ représente un groupe 4-benzyloxyphényle, 4-hydroxyphényle, 2-allyloxyphényle ou 1-naphtyle et $R_1$ représente un groupe alkyle inférieur et $R_2$ le groupe méthyle, ou les mêmes composés à l'état de base libre.

**Revendications pour l'Etat contractant: AT**

1. Procédé pour inverser la configuration de composés énantiomères de formule

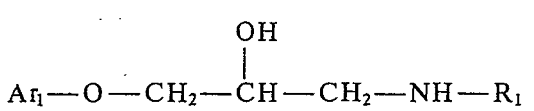

(I)

dans laquelle $Ar_1$ représente un reste mono- ou poly-cyclique carbocyclique ou hétérocyclique éventuellement substitué contenant au moins un cycle à caractère aromatique, et qui est relié à l'atome d'oxygène par l'intermédiaire d'un atome de carbone cyclique, de préference du cycle à caractère aromatique, $R_1$ représente un reste hydrocarboné aliphatique, cycloaliphatique ou araliphatique éventuellement substitué, ou de leurs sels, caractérisé en ce que l'on convertit un énantiomère de formule

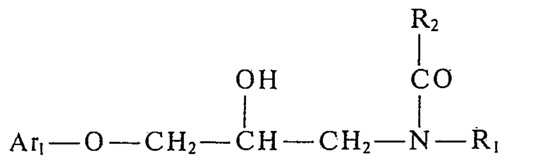

(II)

en configuration R(+) ou S(−) respectivement, $R_2$ représentant un reste mono- ou poly-cyclique carbocyclique ou hétérocyclique éventuellement substitué, ou un reste hydrocarboné aliphatique, cycloaliphatique ou araliphatique éventuellement substitué par traitement à l'aide d'un acide organique ou minéral oxygéné fort ou d'un halogénure d'un tel acide, en un énantiomère de formule

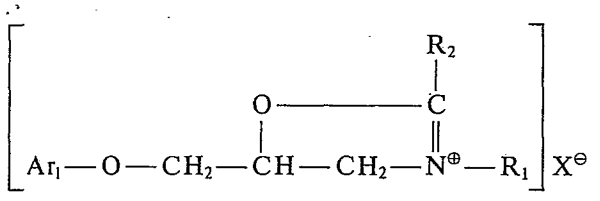

(III)

en configuration R(−) ou S(+) respectivement, $X^{\ominus}$ représentant l'anion d'un acide organique ou minéral oxygéné fort ou un atome d'halogène, et on hydrolyse le composé de formule III ainsi obtenu, en passant le cas échéant par l'intermédiaire de la base libre correspondante, en un composé de formule I dont la configuration est opposée à celle du produit de départ mis en oeuvre, après quoi, si on le désire, on convertit un composé de formule I en un sel ou un sel obtenu en le composé libre.

2. Procédé selon la revendication 1, caractérisé en ce que l'on utilise en tant qu'acides organiques ou minéraux oxygénés forts de l'acide sulfurique concentré, de l'acide phosphorique concentré, de l'anhydride phosphorique ou un acide organique sulfonique fort.

3. Procédé selon la revendication 1, caractérisé en ce que l'on utilise en tant qu'halogénure d'un acide organique ou minéral oxygéné fort le chlorure de thionyle, le bromure de thionyle, le chlorure de sulfuryle, l'acide chlorosulfonique, le chlorure de méthane-sulfonyle, le trichlorure de phosphore, l'oxychlorure de phosphore ou le pentachlorure de phosphore.

4. Procédé selon les revendications 1 et 2, caractérisé en ce que, lorsqu'on utilise un acide organique ou minéral oxygéné fort, on opère dans l'intervalle de température de +50 à +200° C.

5. Procédé selon la revendication 1 ou 3, caractérisé en ce que, lorsqu'on utilise un halogénure d'un acide organique ou minéral oxygéné fort, on opére dans un intervalle de température de −10 à 70° C.

6. Procédé selon la revendication 1, caractérisé en ce que l'on effectue l'hydrolyse en milieu acide ou basique.

7. Procédé selon l'une des revendications 1 à 6, caractérisé en ce que l'on fait réagir un énantiomère de formule II dans laquelle $Ar_1$ représente un groupe naphtyle, alkyle inférieur-phényle, alcényle inférieur-phényle, alcényloxy inférieur-phényle, alcanoylamino inférieur-phényle, alcoxy inférieur-alkyle inférieur-phényle, 9,10-éthano-9,10-dihydro-benzanthryle, pyrazinyle, pyrimidinyle, indolyle, benzofuryle, cyanopyridyle, pyrryl-phényle, alcoxy inférieur-carbonylamino-alkyle inférieur-pyridyle, N'-cycloalkyluréido-phényle, (benzimidazole-2-one)-yle, phényl-alcoxy inférieur-phényle ou hydroxyphényle et $R_1$ représente un groupe alkyle inférieur, phényl-alkyle inférieur, phénoxy-alkyle inférieur ou pyridyloxy-alkyle inférieur, et $R_2$ représente un groupe alkyle inférieur, phényl-alkyle inférieur, phénoxy-alkyle inférieur ou phényle, avec l'acide phosphorique concentré ou l'acide sulfurique concentré ou un halogénure de ces acides, et on hydrolyse le composé de formule III ainsi obtenu, le cas échéant en passant par l'intermédiaire de la base libre correspondante, en le composé correspondant de formule I à configuration opposée.

8. Procédé selon l'une des revendications 1 à 6, caractérisé en ce que l'on fait réagir un énantiomère de départ de formule II dans laquelle $Ar_1$ représente un groupe 1-naphtyle, 3-alkyle inférieur-phényle, 2-alcényle inférieur-phényle, 2-alcényloxy inférieur-phényle, 4-alcanoylamino inférieur-phényle, 4-(2-alcoxy inférieur-éthyl)-phényle, 2-pyrazinyle, 2-pyrimidinyle, (1-pyrryl)-phényle, 4-indolyle, 5-(2-alcoxy inférieur-carbonylamino-éthyl)-pyridyle, 3-cyanopyridyle, 4-(benzimidazole-2-one)-yle, 4-(N'-cycloalkyluréido)-phényle, benzyloxyphényle ou hydroxyphényle et $R_1$ représente un groupe alkyle inférieur, phényl-alkyle inférieur ou phénoxyalkyle inférieur, et $R_2$ représente un groupe alkyle inférieur, phénylalkyle inférieur ou phényle, avec l'acide phosphorique concentré ou l'acide sulfurique concentré ou un chlorure ou bromure de ces acides, et on hydrolyse le composé de formule III ainsi obtenu, le cas échéant en passant par l'intermédiaire de la base libre correspondante, en le composé correspondant de formule I à condiguration opposée.

9. Procédé selon l'une des revendications 1 à 6, caractérisé en ce que l'on fait réagir un énantiomère de formule II dans laquelle $Ar_1$ représente un groupe 1-naphtyle, 3-méthylphényle, 2-allylphényle, 2-allyloxyphényle, 4-acétylaminophényle, 4-indolyle, 4-(2-méthoxyéthyl)-phényle, 2-pyrazinyle, 2-pyrimidinyle, 2-(1-pyrryl)-phényle, 4-(N'-cyclohexyluréido)-phényle, 4-benzyloxyphényle ou 4-hydroxyphényle, $R_1$ représente un groupe alkyle inférieur ou phénylalkyle inférieur et $R_2$ représente un groupe alkyle inférieur, phénylalkyle inférieur ou phényle, avec l'acide phosphorique concentré ou l'acide

sulfurique concentré ou un chlorure ou bromure de ces acides, et on hydrolyse le composé de formule III ainsi obtenu, le cas échéant en passant par l'intermédiaire de la base libre correspondante, à l'aide d'un acide minéral aqueux ou d'une lessive alcaline aqueuse, en le composé correspondant de formule I à configuration opposée.

10. Procédé selon l'une des revendications 1 à 6, caractérisé en ce que l'on fait réagir un énantiomère de formule II dans laquelle $Ar_1$ représente un groupe 2-(1-pyrryl)-phényle, 1-naphtyle, 2-allylphényle, 2-allyloxyphényle, 4-(N'-cyclohexyluréido)-phényle, 2-pyrazinyle, 4-benzyloxyphényle ou 4-hydroxyphényle, $R_1$ représente un groupe alkyle inférieur et $R_2$ un groupe alkyle inférieur ou phényle, avec l'acide sulfurique concentré ou un chlorure de cet acide, ou avec le chlorure de thionyle, et on hydrolyse le composé de formule III ainsi obtenu, le cas échéant en passant par l'intermédiaire de la base libre correspondante, à l'aide d'acide chlorhydrique ou sulfurique aqueux ou à l'aide d'une lessive alcaline aqueuse, en le composé correspondant de formule I à configuration opposée.

11. Procédé de préparation d'énantiomères de formule

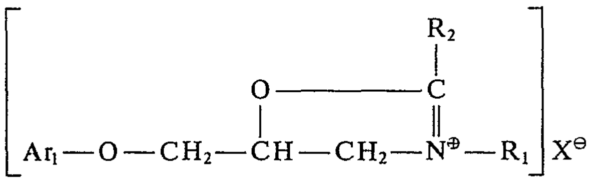

(III)

en condiguration R(−) ou S(+) respectivement, $X^{\ominus}$ représentant l'anion d'un acide organique ou minéral oxygéné fort ou un atome d'halogène et $Ar_1$, $R_1$ et $R_2$ ayant les significations indiquées dans la revendication 1, ce procédé se caractérisant en ce que l'on traite un énantiomère de formule

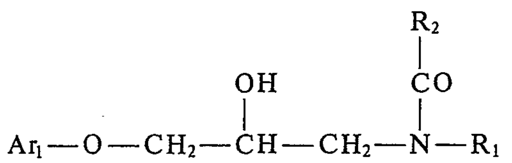

(II)

en configuration R(+) ou S(−) respectivement, $Ar_1$, $R_1$ et $R_2$ ayant les significations indiquées dans la revendication 1, par un acide organique ou minéral oxygéné fort ou un halogénure d'un tel acide.